# FASCICULE DE BREVET EUROPEEN

(11) **EP 0 594 771 B1**
(45) Date de publication et mention de la délivrance du brevet: **03.12.2003**
(21) Numéro de dépôt: 92916151.1
(22) Date de dépôt: 15.07.1992
(51) Int. Cl.: C12N 15/81, C12N 15/65, C12N 1/19

(54) **SEQUENCES NUCLEOTIDIQUES ET PROTEINES DE RESISTANCE A LA CYCLOHEXIMIDE**
NUKLEOTIDSEQUENZEN UND GEGEN CYCLOHEXIMID RESISTENTE PROTEINE
NUCLEOTIDE SEQUENCES AND RESISTANCE PROTEINS CYCLOHEXIMIDE

(30) Priorité: 15.07.1991 FR 9108906
(43) Date de publication de la demande: 04.05.1994
(73) Titulaire: INSTITUT PASTEUR, 75724 Paris Cédex 15 (FR)
(72) Inventeur: DEHOUX, Pierre, F-75006 Paris (FR); DAVIES, Julian, F-75014 Paris (FR)
(74) Mandataire: Desaix, Anne
(86) Numéro de dépôt international: FR9200685
(87) Numéro de publication internationale: WO93002201

(56) Documents cités:
- US-A- 4 857 460
- EMBL DNA DATABASE ACCESSION NR. M94988 9 JUIN 1992, HEIDELBERG, GERMANY P. Dehoux et al. 'Natural cycloheximide resistance in yeast: Role of protein L41'
- EMBL DNA DATABASE ACCESSION NR. M62394 28 Avril 1991, HEIDELBERG, GERMANY S. KAWAI ET AL. 'Kluyveromyces fragilis L41 ribosomal protein (KFL 41) gene, complete cds'

## Description

L'invention concerne des séquences nucléotidiques susceptibles de conférer la résistance à la cycloheximide, des protéines de résistance à la cycloheximide et leur utilisation comme marqueur de sélection, par exemple pour contrôler le transfert d'acides nucléiques.

Les inventeurs ont en effet recherché des marqueurs de sélection adaptés pour contrôler le transfert d'acides nucléiques chez les eucaryotes, marqueurs qui seraient plus efficaces que les marqueurs habituellement utilisés provenant d'organismes procaryotes. On sait par exemple que les marqueurs habituellement utilisés (généticine G-418, hygromycine et bleomycine) proviennent de gènes de résistance isolés chez des procaryotes et sont généralement peu efficaces, notamment chez les champignons. Ces problèmes obligent les expérimentateurs à utiliser de très fortes concentrations en antibiotique, provoquant des problèmes de toxicité et de coût de production. Dès lors d'autres moyens de sélection ont été utilisés, par exemple la sélection par le méthotrexate, la sélection par l'auxotrophie, etc. Cependant ces moyens de sélection ne sont pas applicables de façon générale. Il existe donc un besoin pour la réalisation de systèmes adaptés à la mise en oeuvre d'un marqueur plus efficace et/ou plus puissant que les marqueurs connus jusqu'à présent et d'un coût de production moins onéreux.

La cycloheximide constituerait un marqueur potentiellement intéressant par exemple pour mettre en évidence le transfert d'acide nucléique hétérologue chez des eucaryotes. Dans ce but, les eucaryotes que l'on cherche à modifier de façon contrôlable grâce à un test de détection de la résistance à la cycloheximide, doivent être rendus résistants à cet antibiotique dans des conditions satisfaisantes pour assurer un contrôle fiable du transfert d'acide nucléique hétérologue réalisé soit à des fins de recherche soit dans un but d'exploitation industrielle.

La cycloheximide est un antibiotique qui inhibe la synthèse protéique en se fixant sur la sous-unité 60S ribosomale comme l'ont décrit STOCKLEIN et al (1980, Curr. Genetics 1, 177-183).

Les organismes eucaryotes naturellement résistants à la cycloheximide sont rares; à ce jour des observations relatives aux mécanismes de résistance ont été décrites chez un mutant d'un organisme naturellement sensible à la cycloheximide, le mutant cyh2 de la levure Saccharomyces cerevisiae. Dans ce contexte, le phénomène de résistance est engendré par une modification de la protéine ribosomale L29 (STOCKLEIN et al, 1980).

Ces organismes cellulaires mutants sont généralement résistants à de faibles concentrations en cycloheximide (de l'ordre de 5-10 µg/ml).

D'autres auteurs (TAKAGI et al dans le brevet US 4.857.460) ont rapporté des résultats relatifs à l'étude de la résistance à la cycloheximide chez une autre levure, Candida Maltosa. Ils présentent une séquence d'ADN procurant la résistance à la cycloheximide chez C. maltosa. Cependant aucun gène, aucune phase ouverte de lecture, n'a été identifié dans cette séquence. En d'autres termes le brevet US 4.857.460 ne donne pas les éléments de caractérisation d'un gène, éléments qui auraient pu conduire à définir des modalités pour son utilisation, par exemple pour transformer de façon reproductible des eucaryotes différents de Candida Maltosa, en souches résistantes à la cycloheximide.

Les inventeurs se sont intéressés à la levure Kluyveromyces lactis (K. lactis) et ont mis en évidence le fait que la résistance à la cycloheximide chez K. lactis dépend de l'expression d'un gène particulier. Ils ont aussi identifié le fait que le niveau de résistance de cette levure à cet antibiotique fait intervenir d'autres éléments liés à la présence d'ADN particulier ayant un rôle de cofacteur (ou séquence (s) cofactrice (s)).

Dans un mode de réalisation avantageux de l'invention, la résistance obtenue est spécifique à la cycloheximide, les hôtes transformés résistants à cet antibiotique (transformants cyc^{R}) étant sensibles à plusieurs inhibiteurs classiques des fonctions ribosomales (cryptopleurine, blasticidine, trichodermine, anisomycine, hygromycine).

Les inventeurs ont donc obtenu chez K. lactis une séquence d'acide nucléique d'une taille d'environ 5,2 kb, susceptible de conférer un niveau de résistance à un hôte cellulaire transformé par cette séquence, suffisamment élevé pour la réalisation d'un système de sélection par la résistance à cet antibiotique, cette résistance pouvant se manifester pour une concentration en cycloheximide supérieure à 1 mg/ml.

A l'intérieur de cette séquence d'acide nucléique, les inventeurs ont identifié et caractérisé un gène particulier codant pour une protéine dont la présence s'avère nécessaire pour induire le phénomène de résistance chez K. lactis.

Ce gène particulier code pour une protéine ribosomale qui est responsable de la résistance à une concentration en cycloheximide de l'ordre de 100 µg/ml. La résistance totale à cet antibiotique chez K. lactis nécessite non seulement la présence de cette séquence inductrice de résistance, mais encore la présence d'éléments d'ADN supplémentaires contenus dans la séquence d'acide nucléique de 5,2 kb déterminée par les inventeurs et décrite ci-après. Ces éléments additionnels jouent le rôle de cofacteurs.

La présente demande a donc pour objet des séquences nucléotidiques ainsi qu'une protéine, capables de conférer la résistance à la cycloheximide à un hôte cellulaire eucaryote naturellement sensible à cet antibiotique ou résistant à un bas niveau de concentration (encore désigné par organisme résistant à bas niveau) de cet antibiotique. Ce premier type de protéine pourra être désigné dans la suite par l'expression "protéine de résistance".

L'invention vise aussi une séquence d'acide nucléique comprenant un gène codant pour la protéine de résistance ci-dessus décrite, chez K. lactis, ainsi que des séquences déterminant la présence de cofacteurs impliqués dans le niveau de la résistance conférée par la susdite protéine.

Elle concerne également le gène codant pour la protéine de résistance et les différents fragments d'acide nucléique déterminant la présence des cofacteurs.

Elle vise aussi des vecteurs de clonage et/ou d'expression de ces séquences nucléotiques ainsi que des hôtes cellulaires eucaryotes transformés par ces vecteurs.

Une première protéine selon l'invention ou protéine de résistance à la cycloheximide est une protéine dont la présence est nécessaire et suffisante pour conférer à un hôte naturellement sensible à la cycloheximide, la résistance à cet antibiotique. Elle est caractérisée en ce qu'elle répond à l'enchaînement d'acides aminés A suivant, ou en ce qu'elle comprend tout ou partie de cet enchaînement A éventuellement modifié, dès lors que la protéine formée confère la résistance à la cycloheximide, à un hôte eucaryote recombinant transformé par la séquence nucléotidique codant pour cette protéine, dans des conditions permettant sa production.

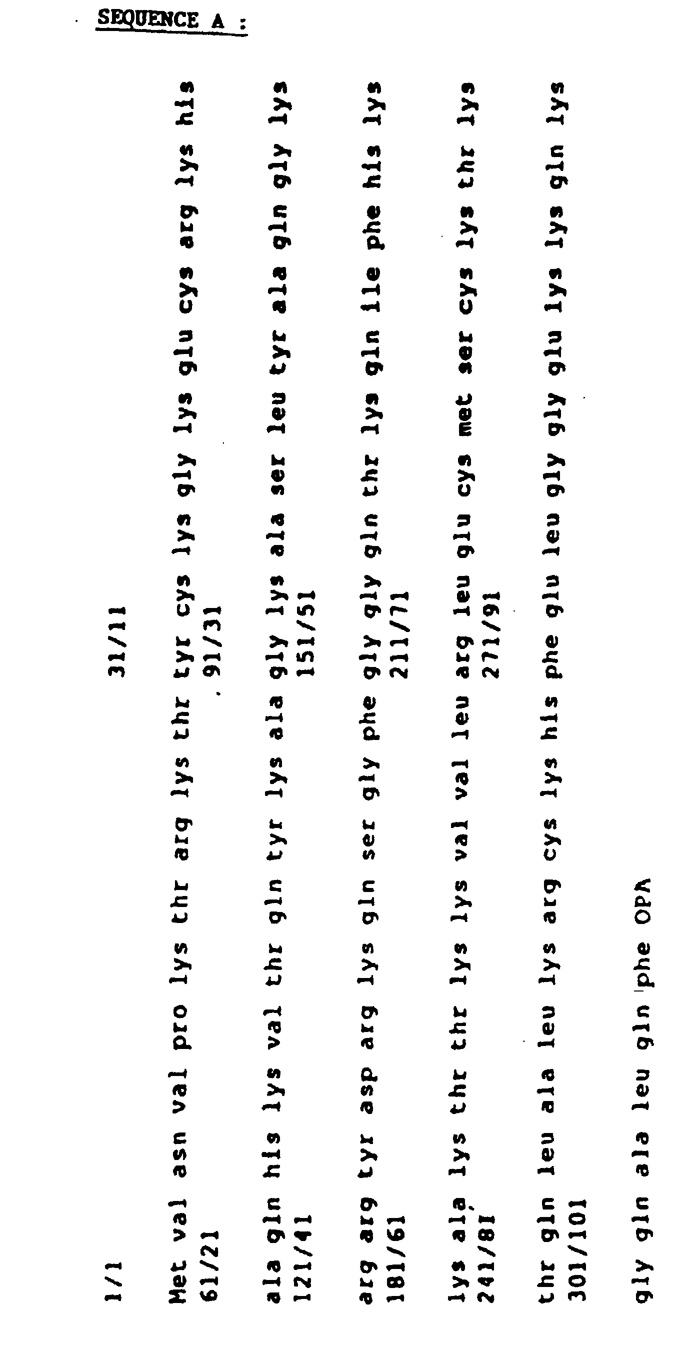

La propriété de cette protéine de conférer la résistance à la cycloheximide peut être évaluée lorsqu'elle est produite dans un hôte cellulaire eucaryote déterminé, naturellement sensible à la cycloheximide à une concentration supérieure à un seuil déterminé en fonction de la nature de l'hôte, et qu'elle permet dans cet hôte d'obtenir une résistance à une concentration supérieure de 5 à 15 fois environ, de préférence de 10 fois environ, par rapport à la concentration de cycloheximide conférant la sensibilité naturelle chez cet hôte.

En particulier chez les levures l'augmentation du niveau de résistance est d'environ 10 fois, voire 100 à 1000 fois chez S. cerevisiae par rapport au niveau observé chez la souche sauvage.

A titre d'exemple on donne ci-dessous un ordre de grandeur de la sensibilité naturelle à la cycloheximide de différents organismes :
levures S : cerevisiae : 1 µg/ml
cellules eucaryotes supérieures : 1 µg/ml
plants de tabac : 10 µg/ml

De façon générale, la sensibilité naturelle d'un organisme déterminé se manifeste dès lors que la présence de cycloheximide dans le milieu de culture inhibe la croissance et la multiplication de l'organisme.

Lorsqu'il est fait référence dans ce texte à un hôte eucaryote sensible à la cycloheximide, cette référence doit être interprétée comme désignant tout organisme eucaryote répondant à la définition ci-dessus s'agissant de la résistance à la cycloheximide.

Une protéine de résistance à la cycloheximide selon l'invention peut encore être caractérisée en ce qu'elle est codée par la séquence nucléotidique I suivante, par une partie de la séquence I ou par une séquence modifiée par rapport à I, dès lors que la protéine codée par cette partie de séquence ou cette séquence modifiée est capable de conférer la résistance à une concentration au moins égale à 100 µg/ml de cycloheximide lorsqu'elle est introduite chez un hôte eucaryote par exemple chez la levure Saccharomyces Cerevisiae, dans des conditions permettant son expression.

Séquence I

AT GGTTAACGTT CCAAAGACCA GAAAGACTTA CTGTAAGGGT
AAGGAGTGCC GTAAGCACGC CCAACACAAG GTTACCCAAT ACAAGGCTGG TAAGGCTTCC
TTGTACGCTC AAGGTAAGAG AAGATATGAC CGTAAACAAT CTGGTTTCGG TGGTCAAACC
AAGCAAATTT TCCACAAGAA AGCTAAGACT ACCAAGAAGG TCGTTTTGAG ATTGGAATGT
ATGTCCTGTA AGACCAAGAC CCAATTGGCT TTGAAGAGAT GTAAGCACTT CGAATTGGGT
GGTGAAAAGA AGCAAAAGGG TCAAGCTTTG CAATTCTGA

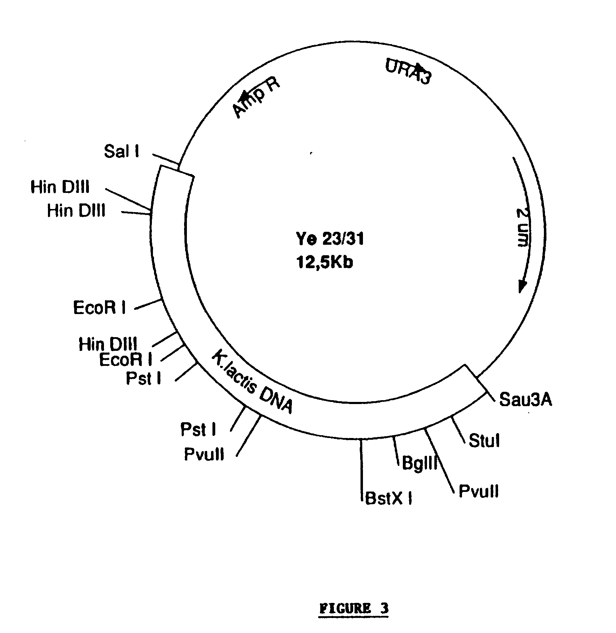

L'invention concerne également une séquence d'acide nucléique comprenant la séquence codant pour la protéine de résistance à la cycloheximide chez K. lactis, cette séquence étant caractérisée en ce qu'elle répond à l'une des définitions suivantes :
(a) il s'agit d'une séquence d'acide nucléique de K. lactis d'environ 5,2 kb, capable de conférer une résistance à la cycloheximide à une concentration supérieure à 1 mg/ml chez K. lactis ou chez S. cerevisiae, et comprenant les sites de restriction définis à la figure 3; Les divers sites sont situés respectivement aux positions suivantes :
   Sau3A : 0, StuI : +300 bp, PvuII : +600 bp, BglII : +900 bp, BstXI : +1200 bp, PvuII : +2300 bp, Pst I : +2400 bp, PstI : +3000 bp, EcoRI : +3200 bp, HindIII : +3300 bp, EcoRI : +3700 bp, HindIII : +4500 bp, HindIII : +4540 bp, SalI : +5100 bp.
(b) il s'agit d'une séquence d'acide nucléique d'environ 5,2 kb, comprenant l'enchaînement nucléotidique I ou hybridant avec la séquence complémentaire de l'enchaînement I, dans des conditions de forte stringence (0,1 X SSC, 0,1% SDS à 65°C), et capable de conférer une résistance à une concentration de cycloheximide supérieure à 100 µg/ml, de préférence supérieure à 1 mg/ml, chez K. lactis;
(c) il s'agit d'une séquence d'acide nucléique d'environ 5,2 kb comprise dans l'enchaînement nucléotidique II suivant ou hybridant avec la séquence complémentaire de l'enchaînement II, dans des conditions de forte stringence, et capable de conférer une résistance à une concentration de cycloheximide supérieure à 100 µg/ml, de préférence supérieure à 1 mg/ml, chez K. lactis;
(d) il s'agit de la séquence d'acide nucléique comprenant la séquence de nucléotides située entre les positions 9 et 2763 de l'enchaînement II ou une séquence hybridant avec la séquence complémentaire de l'enchaînement II, dans des conditions de forte stringence, et capable de conférer une résistance à une concentration de cycloheximide supérieure à 100 µg/ml, de préférence supérieure à 1 mg/ml, chez K. lactis.
Lorsqu'une séquence d'acide nucléique répondant à l'une des définitions données ci-dessus est introduite chez une levure telle que S. cerevisiae, elle confère à cette levure, une résistance à la cycloheximide à une concentration supérieure à 100 µg/ml, cette résistance

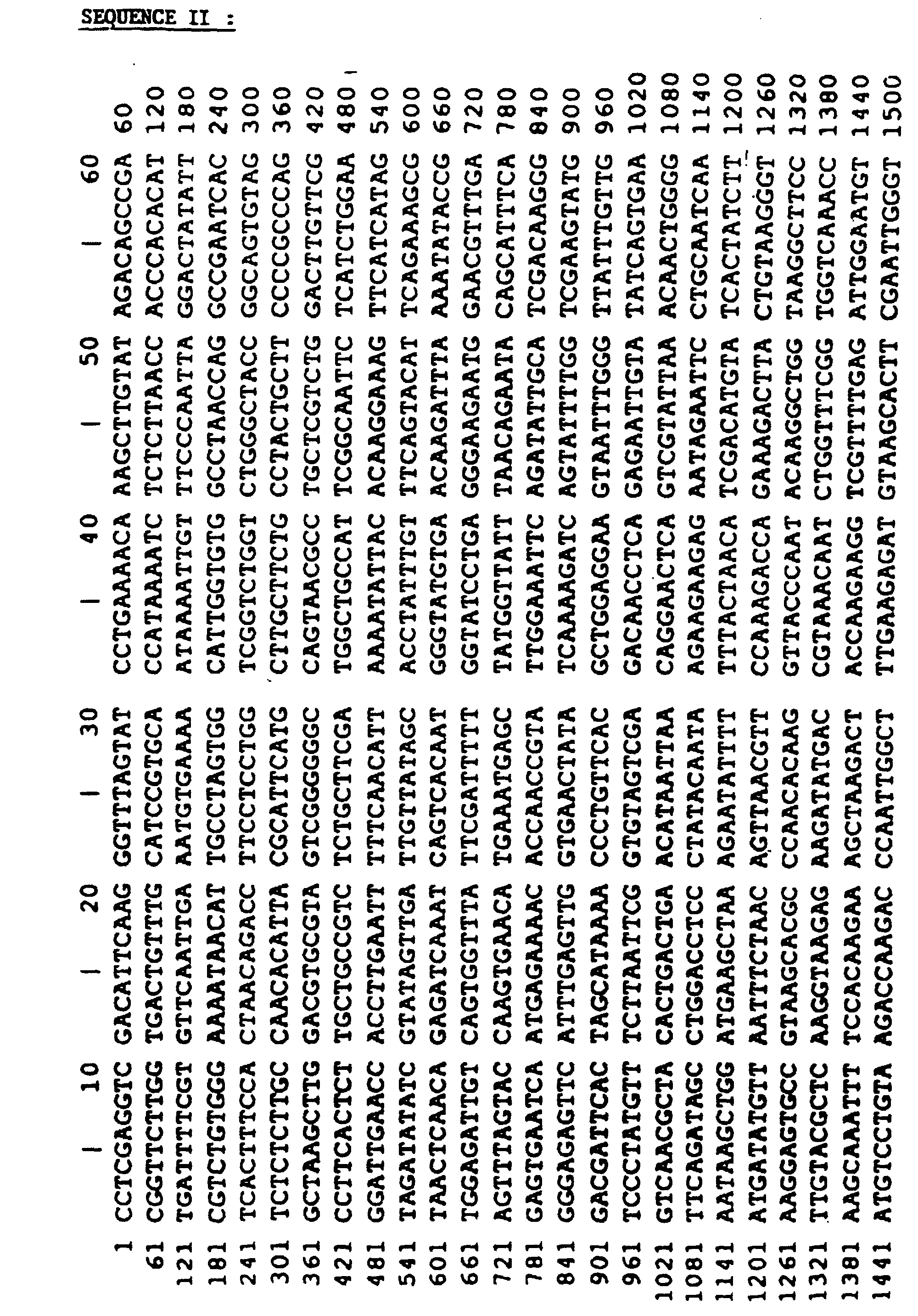

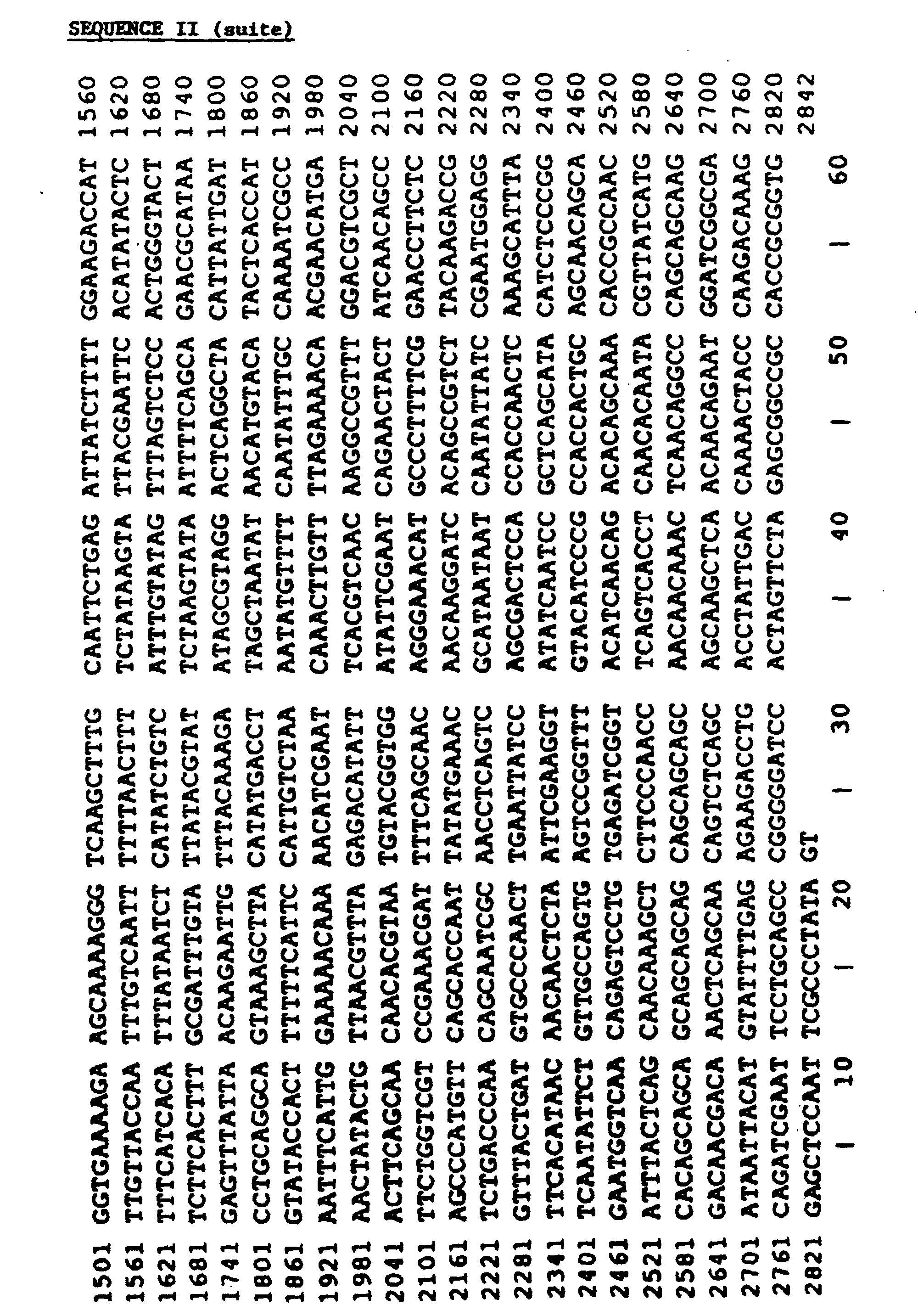

se manifestant avantageusement pour une concentration en cycloheximide supérieure à 1 mg/ml.

Selon un mode de réalisation préféré de l'invention, les deux séquences d'ADN définies aux points (b) et (c) ci-dessus, répondent en outre à la carte de restriction donnée à la figure 3.

Selon un autre mode de réalisation préféré de l'invention, la séquence d'acide nucléique ci-dessus décrite est extraite de la souche E.coli DH5α recombinée avec le plasmide Ye23/31, déposée à la CNCM (Collection Nationale de Culture de Microorganismes à Paris, France) le 2 Juillet 1991 sous le numéro I-1121.

On appele ici "séquence complémentaire" d'une séquence d'acide nucléique donnée, une séquence inverse et complémentaire par rapport à une séquence donnée. Le terme "inverse" rend compte de la restauration de l'orientation 5' - 3' de l'acide nucléique qui est aussi complémentaire par la nature des nucléotides qu'elle contient, à une séquence donnée.

La séquence d'acide nucléique de 5,2 kb comprend, outre la séquence codant pour la protéine dite de résistance, ci-dessus décrite, l'ADN que l'on peut désigner par le terme "cofacteur", qui détermine le niveau de résistance à la cycloheximide chez K. lactis et chez un hôte cellulaire transformé par cette séquence. Cet ADN cofacteur est compris dans la séquence II.

L'invention concerne donc également les fragments d' ADN suivants, inclus dans la séquence de 5, 2 kb ci-dessus décrite :
le fragment BamHI (5') - PvuII (3') d' environ 2,3 kb
le fragment PvuII (5') - SalI (3') d'environ 3 kb

L'obtention d'une résistance à la cycloheximide chez un hôte cellulaire donné, à un niveau déterminé peut être réalisée en transformant cet hôte avec un acide nucléique codant pour la protéine de résistance à la cycloheximidee et avec un fragment d'ADN cofacteur présent dans la séquence de 5,2 kb adjacent ou non à l'acide nucléique ci-dessus, l'introduction de ce fragment chez l'hôte étant directement corrélée à l'importance de la résistance constatée. Un mode de réalisation de la transformation d'un hôte cellulaire est décrit dans les pages suivantes.

La protéine de résistance peut être isolée par lyse des cellules de K. lactis suivie des étapes décrites dans "The Yeasts" (seconde édition par A.H. Rose et J.S. Harrison, vol 4: 504-505).

La purification peut aussi être faite par affinité au moyen d'anticorps digérés contre la séquence A. Dans ce cas on réalise une surexpression de la protéine ribosomale qui est ensuite purifiée selon les techniques classiques, sur gel de polyacrylamide. La bande de poids moléculaire correspondant à la protéine de séquence A est prélevée. Cette bande est la bande majoritaire pusiqu'elle résulte de la surexpression de la protéine. Elle est soumise à une réaction avec les anticorps ci-dessus décrits, produits selon les techniques classiques, s'agissant notamment d'anticorps monoclonaux.

L'invention vise aussi la séquence ORF A contenant l'acide nucléique I, ORF A étant elle même comprise entre les nucléotides en positions 1 et 1560 de l'enchaînement II étant entendu que la séquence comprise entre les nucléotides 633 et 1222 correspond à un intron V. La séquence ORF A comporte deux exons, l'un étant formé par les nucléotides ATGG situés entre les nucléotides 629 et 632, l'autre exon étant formé par la séquence comprise entre les nucléotides 1223 et 1539.

L'enchaînement nucléotidique compris entre les nucléotides 1 et 1539 comporte, outre la séquence ORF A, le promoteur du gène.

D'autres séquences nucléotidiques particulièrement intéressantes dans le cadre de l'invention sont les séquences suivantes :
- l'enchaînement nucléotidique ORF A en tant que tel ou tout enchaînement nucléotidique hybridant avec la séquence complémentaire de ORF A dans les conditions suivantes : 60°C, 2 x SSC, 5 x Denhart, 0,1% SDS, 0,1 mg/ml d'ADN de sperme de saumon,
- la séquence codante I (ou phase exonique) contenue dans l'acide nucléique ORF A codant pour la protéine de résistance chez Kluyveromyces lactis, et comprise entre les nucléotides 629 et 1539, formant une séquence de deux exons dont l'un est compris entre les nucléotides 629 et 632 et l'autre entre les nucléotides 1223 et 1539,
- la séquence IV, comprenant les nucléotides 1561 et 2740 et correspondant à l'enchaînement situé entre les nucléotides 1540 et 2763 appelée cofacteur et capable d'augmenter la résistance à la cycloheximide conférée par la protéine de résistance,
- la phase intronique V de ORF A,
- l'enchaînement nucléotidique III délimité par les nucléotides 1 et 628 plus particulièrement 8 et 628 qui contient les éléments de régulation de l'expression de ORF A et en particulier la région promotrice de la transcription de la séquence ORF A; ce promoteur contient en particulier des régions régulatrices des promoteurs de protéines caractéristiques des ribosomes, régions de type "UAS_{RPG}" telles que décrites par W.H. Mager (Biochem. Biophys. Acta (1988) 949: 1-15). Ces protéines dites ribosomales sont importées dans le noyau de la cellule qui les produit, où elles sont assemblées en ribosome,
- la séquence IV comprise entre les nucléotides 1561 et 2740 appelée cofacteur et capable d'augmenter la résistance à la cycloheximide conférée par la protéine de résistance.

L'invention a aussi pour objet des vecteurs recombinants en particulier pour le clonage et/ou l'expression des protéines dont on a précédemment donné les caractéristiques.

Ces vecteurs d'expression sont caractérisés par l'incorporation en un de leurs sites non essentiels pour leur réplication, d'une séquence nucléotidique choisie parmi les précédentes, sous le contrôle des éléments de régulation nécessaires à son expression dans un hôte cellulaire eucaryote choisi, ces éléments de régulation comportant notamment un promoteur, le cas échéant inductible et une séquence de terminaison de transcription.

Un tel vecteur peut comprendre une séquence nucléotidique codant pour une protéine dite protéine de résistance à la cycloheximide, seule ou en association avec une séquence nucléotidique cofactrice répondant à la définition donnée précédemment.

L'invention vise en outre des vecteurs répondant aux définitions ci-dessus caractérisés en ce qu'ils contiennent en outre un acide nucléique hétérologue déterminé, par exemple un acide nucléique codant pour une protéine d'intérêt pharmaceutique ou industriel, cet acide nucléique étant sous le contrôle d'éléments de régulation nécessaires à son expression dans l'hôte, ces éléments pouvant être confondus avec les séquences contrôlant la transcription des séquences nucléotidiques intervenant dans la résistance à la cycloheximide.

Le système de résistance selon l'invention permet de façon avantageuse de contrôler l'insertion de séquences hétérologues telles celle de la Serum Albumine Humaine, de l'antigène de surface de l'hépatite B, en vue de leur expression dans un hôte eucaryote.

A titre d'exemple on peut utiliser des vecteurs du type plasmide réplicatif, des vecteurs intégratifs tels que pSVL (Pharmacia). Un tel vecteur peut par exemple contenir la séquence nucléotidique codant pour la protéine répondant à la séquence A, dépourvue de son intron.

De façon générale, il est intéressant pour la mise en oeuvre de l'invention, d'utiliser des vecteurs à multiples copies en particulier quand les cellules transformées sont des cellules eucaryotes.

Des vecteurs préférés selon l'invention sont des vecteurs adaptés à l'expression dans des levures notamment d'intérêt industriel que l'on souhaite marquer avec un marqueur de résistance par exemple lorsqu'on ne dispose pas d'un marqueur d'auxotrophie, une levure particulière étant Pichia pastoris ou S. pombe, ou des vecteurs adaptés à l'expression dans une cellule animale d'eucaryote supérieur, par exemple le baculovirus, ou encore des vecteurs adaptés à l'expression dans une cellule végétale telle que les plants de tabac. Ainsi des cellules de singe , ou des cellules de souris peuvent être transformées.

Un vecteur particulièrement avantageux dans le cadre de l'invention est le plasmide Ye23/31 transformant la souche E.coli DH5α déposée à la CNCM le 2 Juillet 1991 sous le numéro I-1121.

L'invention vise par ailleurs une cellule eucaryote caractérisée en ce qu'elle est transformée par un vecteur répondant aux caractéristiques ci-dessus et en particulier en ce qu'il s'agit d'une cellule de levure, par exemple Pichia pastoris, d'une cellule animale d'eucaryote supérieur, par exemple une cellule d'insecte ou une cellule de mammifère notamment murine ou simienne, d'une cellule humaine ou encore d'une cellule végétale.

Une telle cellule recombinante peut être obtenue par tous les procédés d'insertion de séquences hétérologues couramment utilisés pour la préparation d'hôtes cellulaires recombinants. On peut notamment appliquer la technique d'électroporation décrite par M. Becker, L. Garente dans Method in Enzymology (1991 Vol. 194: 182-187).

On pourra également avoir recours aux techniques décrites dans la demande de brevet WO 84/02913 au sujet de l'insertion dans des cellules végétales, de séquences nucléotidiques hétérologues par rapport aux séquences d'acides nucléiques naturellement contenues dans de telles cellules.

A titre d'application, les protéines de résistance décrites précédemment peuvent être utilisées dans un système de sélection répondant spécifiquement à un marqueur de type cycloheximide, pour contrôler l'introduction chez un hôte eucaryote d'une séquence hétérologue que l'on souhaite exprimer dans cet hôte. La sélection sera d'autant plus aisée que la résistance peut être vérifiée avec une forte concentration d'antibiotique cette résistance résultant alors de l'association à la protéine de résistance ci-dessus à un cofacteur défini précédemment.

Il est particulièrement intéressant dans le cadre de l'invention de préparer des protéines permettant d'obtenir une résistance à une concentration d'environ 100 µg/ml, voire 1 mg/ml de cycloheximide. Il est en outre dans certains cas très avantageux d'obtenir des protéines de résistance éventuellement en association avec un cofacteur conduisant à une résistance à une concentration d'environ 1 mg/ml de cycloheximide.

L'invention vise par ailleurs un procédé pour le contrôle de la présence d'un acide nucléique hétérologue dans un hôte cellulaire, caractérisé en ce qu'il comprend :
- la transformation de l'hôte cellulaire par un vecteur d'expression comportant en des sites non essentiels pour sa réplication, l'acide nucléique hétérologue d'une part, une séquence nucléotidique codant pour une protéine de résistance d'autre part et le cas échéant une séquence nucléotidique cofactrice sous le contrôle des éléments de régulation nécessaires à l'expression de ces séquences dans l'hôte cellulaire choisi,
- la culture de l'hôte cellulaire ainsi transformé,
- la mise en contact de l'hôte avec une concentration déterminée de cycloheximide et la détection de la résistance de l'hôte vis à vis de cet antibiotique.

L'invention a aussi pour objet une méthode pour l'obtention de cellules exprimant la résistance à la cycloheximide telle que décrite dans les pages précédentes caractérisée par les étapes suivantes :
- transformation d'un hôte cellulaire donné, par une séquence d'acide nucléique de l'invention préalablement insérée dans un plasmide de l'hôte, dans des conditions permettant l'expression de le susdite séquence d'acide nucléique;
- culture des cellules transformées (transformants) dans un milieu complet contenant une concentration de cycloheximide très faible, de préférence inférieure à 2 µg/ml;
- récupération des souches résistantes à une concentration supérieure à 1 à 10 µg/ml en cycloheximide.

A titre d'exemple, s'agissant des levures, les étapes décrites ci-dessus sont effectuées dans les conditions suivantes :
- la culture des transformants est réalisée à une température comprise entre environ 29°C et environ 30°C, de préférence 30°C, en présence d'une concentration en cycloheximide inférieure à 2 µg/ml, pendant 12 à 36 heures,
- les souches sélectionnées à partir de cette culture sont celles qui résistent en culture en présence de 100 µg/ml de cycloheximide,
- le milieu complet de culture contient 10 g/l d'extrait de levure (yeast extract), 20 g/l de Bacto peptone et 20 g/l de glucose.

De préférence l'étape de culture des transformants dont il est question ci-dessus est réalisée pendant une durée de 12 à 18 heures à 30°C, après transformation dans un milieu complet liquide à des concentrations sub-limitantes (1 µg/ml). Cette concentration peut être adaptée en fonction de la souche et de l'espèce de levure utilisées.

Pour des cellules eucaryotes, comme par exemple les cellules LTK- (souris) le milieu de culture peut être un milieu Dulbeco modifié par 10% de sérum de veau nouveau né et par un antibiotique fongicide.

Les clones recombinants sont sélectionnés selon la technique décrite par Delpeyroux F. et al dans Journal of Virology (Dec 1990 Vol. 64 (12): 6090-6100).

En résumé, les cellules sont cotransfectées avec 10 fois plus de plasmide recombinant que de vecteur contenant le gène de résistance à l'antibiotique G418 (Colbère - Garapin F. et al 1981, J. Mol. Biol 150: 1-14). Après trois semaines, les clones résistants au G418 sont mis en milieu de culture additionné de cycloheximide à une concentration de 1 à 10 µg/ml.

Lorsque l'hôte cellulaire est S. cerevisiae, le plasmide contenant la séquence nucléotidique de l'invention peut être un plasmide multicopie épisomal de la famille des plasmides YEp, par exemple le plasmide 2µ de S. cerevisiae, ou le plasmide multicopie épisomal Ye 23/31.

D'autres avantages et caractéristiques de l'invention ressortent des exemples et des figures qui suivent.

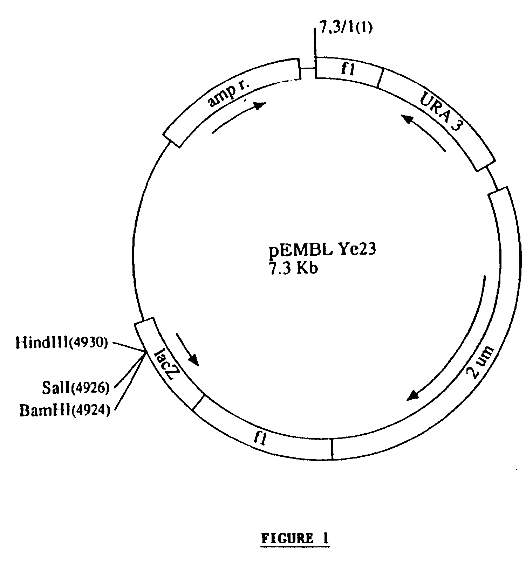

### Figure 1 : vecteur de clonage contenant le fragment d'ADN de K. lactis d'environ 5,2 kb procurant la résistance à la cycloheximide

Une banque génomique de la souche de K. lactis 2359-152 résistante à plusieurs mg/ml de cycloheximide a été construite. Cette banque est réalisée dans un vecteur navette E.coli/S. cerevisiae :pEMBL Ye 23 (Baldari et al 1985 Gene 35: 27-32, figure 1). L'ADN chromosomique de K. lactis a été digéré partiellement par l'enzyme Sau3A puis fractionné sur un gradient de sucrose. Les fractions contenant un ADN de taille comprise entre 4 et 9 kb ont été rassemblée et l'ADN ligaturé au vecteur Ye 23 préalablement coupé et déphosphorylé au site BamHI situé dans le gène de la β-galactosidase. Ce mélange de ligation a permis de transformer la souche de E.coli XL1 et les transformants recombinants ont été sélectionnés sur un milieu permettant de visualiser une insertion dans le gène de β-galactosidase. 23000 clones de E.coli recombinants ont été ainsi sélectionnés. L'analyse par restriction des plasmides de cinquante clones a permis d'estimer la taille moyenne de l'insertion d'ADN de K. lactis à 5 kb, ce qui donne une probablité de 99,5% d'obtenir un gène spécifique.

Souche de E.coli XL1 (souche mise au point par la société Stratagène : endAl, hsdRl7 (rk⁻, mk⁺), supE44, thi⁻, λ⁻, recAl, gyrA96, relAl, Δ (lac), F', proAB, laciq ZΔM15, Tn10 (tet^{r})

Souche de S. cerevisiae OL1: α, leu2-3, leu2-112, his3-11, his3-15, ura3-251, ura3-273

### Figure 2 : Fragment séquencé de l'ADN de K. lactis procurant la résistance à la cycloheximide (séquence II)

### Figure 3 : carte de restriction du fragment d'ADN de 5,2 kb de K. lactis qui a été inséré au site BamHI du vecteur Ye 23 (C. Baldari et al, Gene (1985) 35: 27)

Le fragment SalI-PvuII (2,8 kb) séquencé contient la protéine ribosomale. Les divers sites sont situés respectivement aux positions suivantes :
Sau3A : 0, StuI : +300 bp, PvuII : +600 bp, BglII : +900 bp, BstXI : +1200 bp, PvuII : +2300 bp, Pst I : +2400 bp, PstI : +3000 bp, EcoRI : +3200 bp, HindIII : +3300 bp, EcoRI : +3700 bp, HindIII : +4500 bp, HindIII : +4540 bp, SalI : +5100 bp.

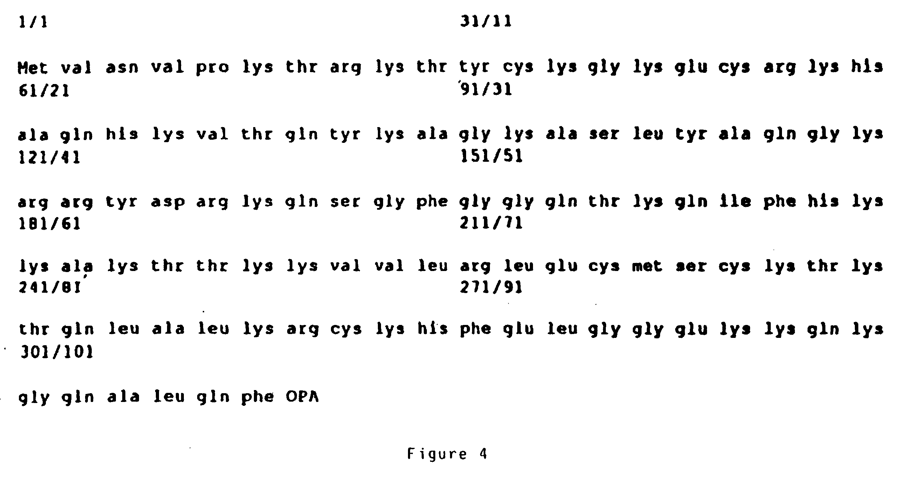

### Figure 4 : Protéine conférant la résistance à la cycloheximide, codée par l'ADN de K. lactis

### EXEMPLES

### I - Clonage du fragment d'ADN de K.lactis procurant la résistance à la cycloheximide

### I.1) Construction d'une banque de K.lactis chez S.cerevisiae :

Une banque génomique de la souche de K.lactis 2359-152 (WESOLOWSKI et al, 1982, Curr. Genetics. 5: 191-197) résistante à des concentrations de plusieurs mg/ml de cycloheximide a été construite. Cette banque a été réalisée dans un vecteur navette E.coli/S.cerevisiae : pEMBL Ye 23 (BALDARI et al, 1985, Gene 35: 27-32), (figure 1). L'ADN chromosomique de K.lactis a été digéré partiellement par l'enzyme Sau3A puis fractionné sur un gradient de sucrose. Les fractions contenant un ADN de taille comprise entre 4 et 9 kb ont été rassemblées et ligaturées au vecteur Ye 23 préalablement coupé et déphosphorylé au site BamHI situé dans le gène β-galactosidase. Ce mélange de ligation a permis de transformer la souche de E.coli XL1 (BULLOCK et al, 1987, Biotechniques 5, 376-379) et les transformants recombinants ont été sélectionnés sur un milieu permettant de visualiser une insertion dans le gène de la β-galactosidase. 23000 clones de E.coli recombinants ont été sélectionné. L'analyse par restriction des plasmides de cinquante clones a permis d'estimer la taille moyenne de l'insertion d'ADN de K.lactis à 5,2 kb, ce qui donne une probabilité de 99,5% d'obtenir un gène spécifique.

### I.2) Transformation de S.cerevisiae et obtention de résistants à la cycloheximide :

La souche OL1 de S.cerevisiae (BOY-MARCOTTE et al, 1982, Gene 20: 433-440), sensible à des concentrations inférieures à 1 µg/ml de cycloheximide a été transformée avec l'ADN extrait de la banque. Dans un premier temps les transformants ont été criblés pour la prototrophie uracil ce qui a permis d'évaluer la quantité totale de transformants. Dans un deuxième temps nous avons répliqué ces transformants sur un milieu contenant de la cycloheximide respectivement à deux concentrations : 10 et 100 µg/ml. 35000 transformants URA⁺ ont été obtenus dont 7 sont résistants à des concentrations supérieures à 100 µg/ml de cycloheximide. Après analyse, il s'est avéré que 6 parmi les 7 clones retenus avaient intégré l'ADN provenant de K.lactis dans leur génome. Cependant, chez le septième transformant, le caractère de résistance a été maintenu sur le plasmide Ye 23/31 dérivé du vecteur pEMBLYe23. Ce transformant est résistant à de très fortes concentrations de cycloheximide dépassant 1 mg/ml. Le fragment d'ADN procurant la résistance à la cycloheximide est d'environ 5,2 kb.

D'autre part, pour les transformants ayant intégré dans leur génome le fragment d'ADN de K.lactis, le phénomène de résistance est maintenu chez un diploïde hétérozygote pour ce marqueur. Le caractère "cycloheximide résistant" est donc dominant.

### I.3) Protéine responsable de la résistance à la cycloheximide :

La totalité de ce fragment a été insérée dans des vecteurs phagemides pBluescript (Stratagene). Différentes délétions unidirectionnelles ont été réalisées à l'aide du couple d'enzymes ExoIII/S1. Le séquençage de l'ADN simple brin a été effectué à l'aide la technique de Sanger en utilisant des amorces universelles ou des amorces synthétisées au laboratoire à partir de la séquence elle même.

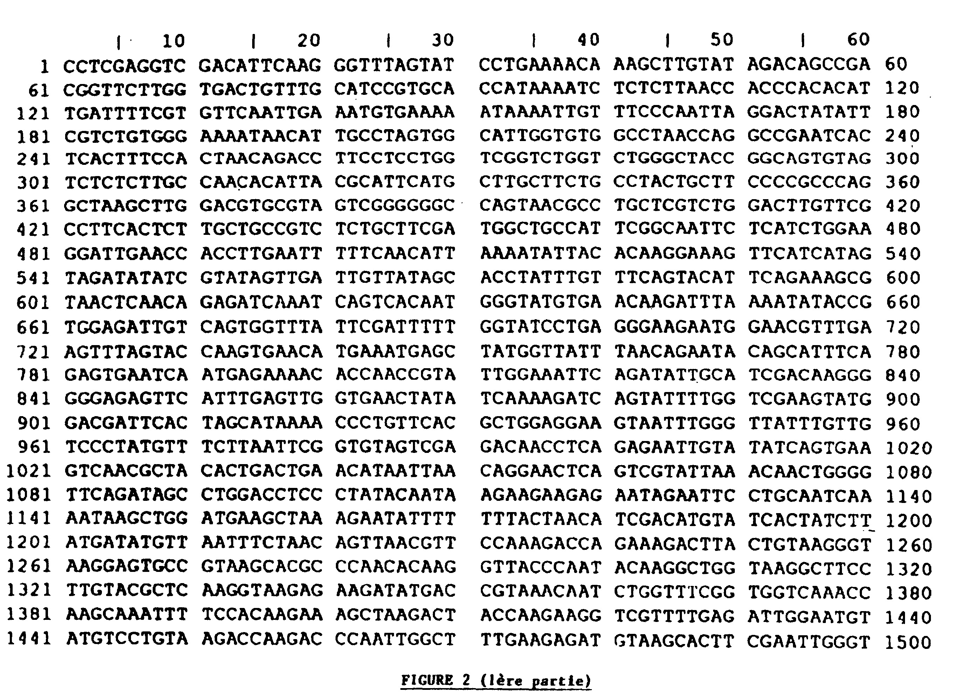

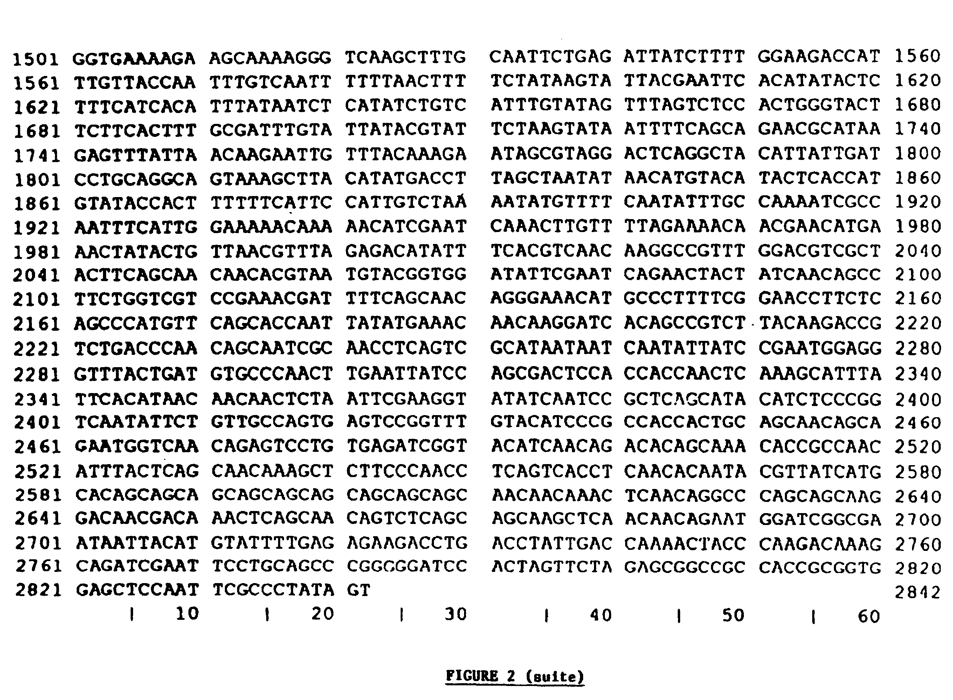

Un fragment de la séquence ainsi obtenue est présenté figure 2. Elle contient notamment une phase ouverte de lecturee ORF A.

### Phase ORF A :

Cette phase de lecture est longue de 320 bp (figure 2) et elle est dotée d'un intron de 590 bp. Cet intron est localisé à l'extrémité 5' du gène, après la première base après l'ATG. La protéine codée par l'exon est de 150 acides aminés (figure 4). La recherche d'homologies à l'aide des banques de données EMBL et GENEBANK montre que l'ORF A est homologue à 75% au niveau des acides aminés à la protéine ribosomale L36a du rat (GALLAGHER et al, 1988, DNA7: 269-273) et à 74% à une protéine ribosomale humaine (DAVIES et al, 1986, Gene 45: 183-191) qui est elle même homologue à la protéine ribosomale rp44 (aussi dénomée L41) de S.cerevisiae (ITOH, 1978 FEBS Lett. 96: 399-402). L

Cette séquence est suffisante pour procurer un niveau de résistance élevé vis à vis de la cycloheximide, cependant ce niveau est plus faible que celui observé chez les transformants possédant le fragment initial de 5,2 kb.

### I.4) Expression de la phase de lecture A :

Afin de déterminer dans quelle mesure la protéine dite de résistance est responsable du phénomène de résistance à la cycloheximide, la même souche de S.cerevisiae a été transformée dans les mêmes conditions avec un vecteur contenant uniquement l'ORF A. Les transformants qui possèdent l'ORF A sont résistants à la cycloheximide. Cependant le niveau de résistance observé (100 µg/ml) est environ dix fois inférieur à celui obtenu lorsque ORF A est associée avec l'ADN cofacteur contenu dans le fragment total de 5,2 kb (1000 µg/ml). Par contre la délétion de la séquence ORF A entraîne l'abolition de la résistance.

### Conclusions :

Un gène responsable du phénomène de résistance à la cycloheximide a été isolé sur un fragment d'ADN de K.lactis. Ce système est composé d'une protéine ribosomale (ORF A) très fortement analogue aux protéines ribosomales L36a humaine et de rat ainsi que la rp44 (ou L41) de S. cerevisiae. Cette protéine est nécessaire pour provoquer le phénomène de résistance mais non suffisante pour avoir une résistance totale vis à vis de l'antibiotique. La présence d'un cofacteur localisé sur le fragment clone de 5,2 kb de K lactis est nécessaire pour obtenir une résistance à un niveau de 1 mg/ml environ chez K lactis.

## Revendications

1. séquence d'acide nucléique de K. lactis d'environ 5,2 kb, capable de conférer une résistance à la cycloheximide à une concentration supérieure à 1 mg/ml chez K. lactis ou chez S. cerevisiae et comprenant les sites de restriction : Sau3A : 0, StuI : +300 bp, PvuII : +600 bp, BglII : +900 bp, BstXI : +1200 bp, PvuII : +2300 bp, Pst I : +2400 bp, PstI : +3000 bp, EcoRI : +3200 bp, HindIII : +3300 bp, EcoRI : +3700 bp, HindIII : +4500 bp, HindIII : +4540 bp, SalI : +5100 bp.

2. séquence d'acide nucléique d'environ 5,2 kb selon la revendication 1, comprenant l'enchaînement nucléotidique I suivant : 
Séquence I

AT GGTTAACGTT CCAAAGACCA GAAAGACTTA CTGTAACGGT
AACGAGTGCC GTAAGCACGC CCAACACAAG GTTACCCAAT ACAAGGCTGG TAAGGCTTCC
TTCTACGCTC AAGGTAAGAG AAGATATGAC GGTAAACAAT CTGGTTTCGG TGGTCAAACC
AAGCAAATTT TCCACAAGAA AGCTAAGACT ACCAAGAAGG TCGTTTTGAC ATTGGAATGT
ATGTCCTCTA ACACCAAGAC CCAATTGCCT TTGAACACAT GTAAGCACTT CGAATTGGGT
GGTGAAAAGA AGCAAAAGGC TCAAGCTTTG CAATTCTGA
 ou hybridant avec la séquence complémentaire de l'enchaînement I, dans des conditions de forte stringence (0,1 x SSC, 0,1% SDS à 65°C), et capable de conférer une résistance à la cycloheximide à une concentration supérieure à 100 µg/ml, de préférence supérieure à 1 mg/ml, chez K. lactis.

3. Séquence d'acide nucléique d'environ 5,2 kb selon la revendication 1 ou 2, comprenant l'enchainement nucléotidique II suivant : 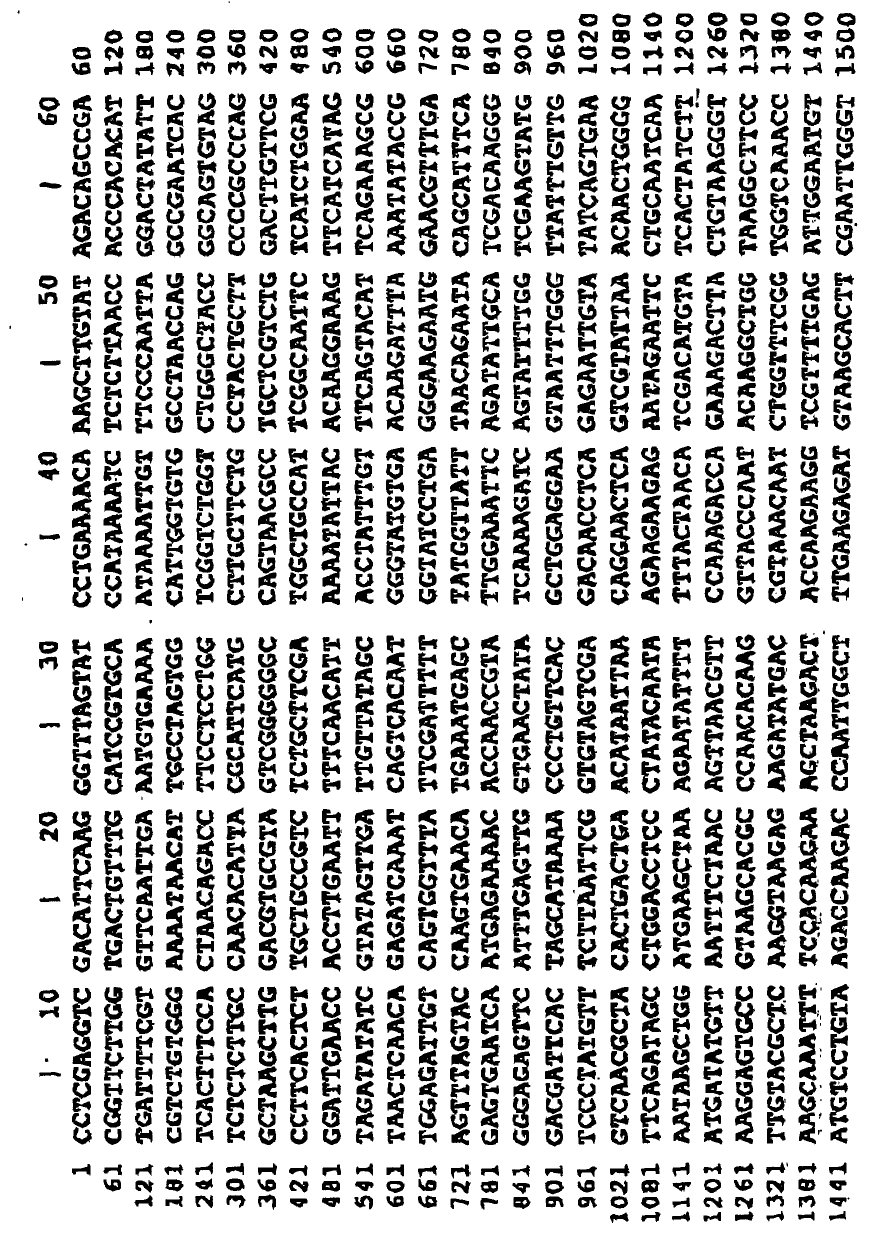 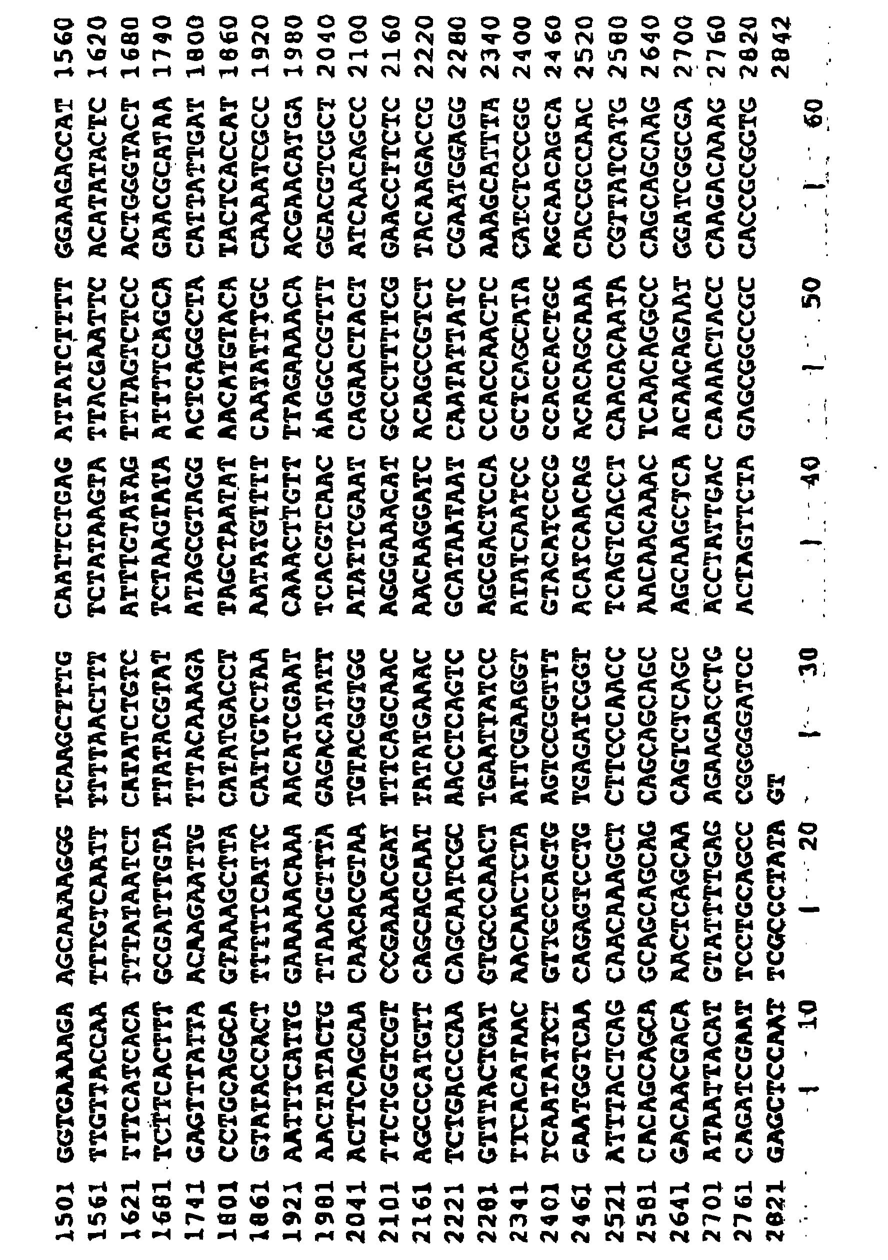 ou hybridant avec la séquence complémentaire de l'enchaînement II dans des conditions de forte stringence, et capable de conférer une résistance à une concentration de cycloheximide supérieure à 100 µg/ml, de préférence supérieure à 1 mg/ml, chez K. lactis.

4. séquence d'acide nucléique selon la revendication 1 ou 2, comprenant la séquence de nucléotides située entre les positions 9 et 2763 de l'enchaînement II suivant : 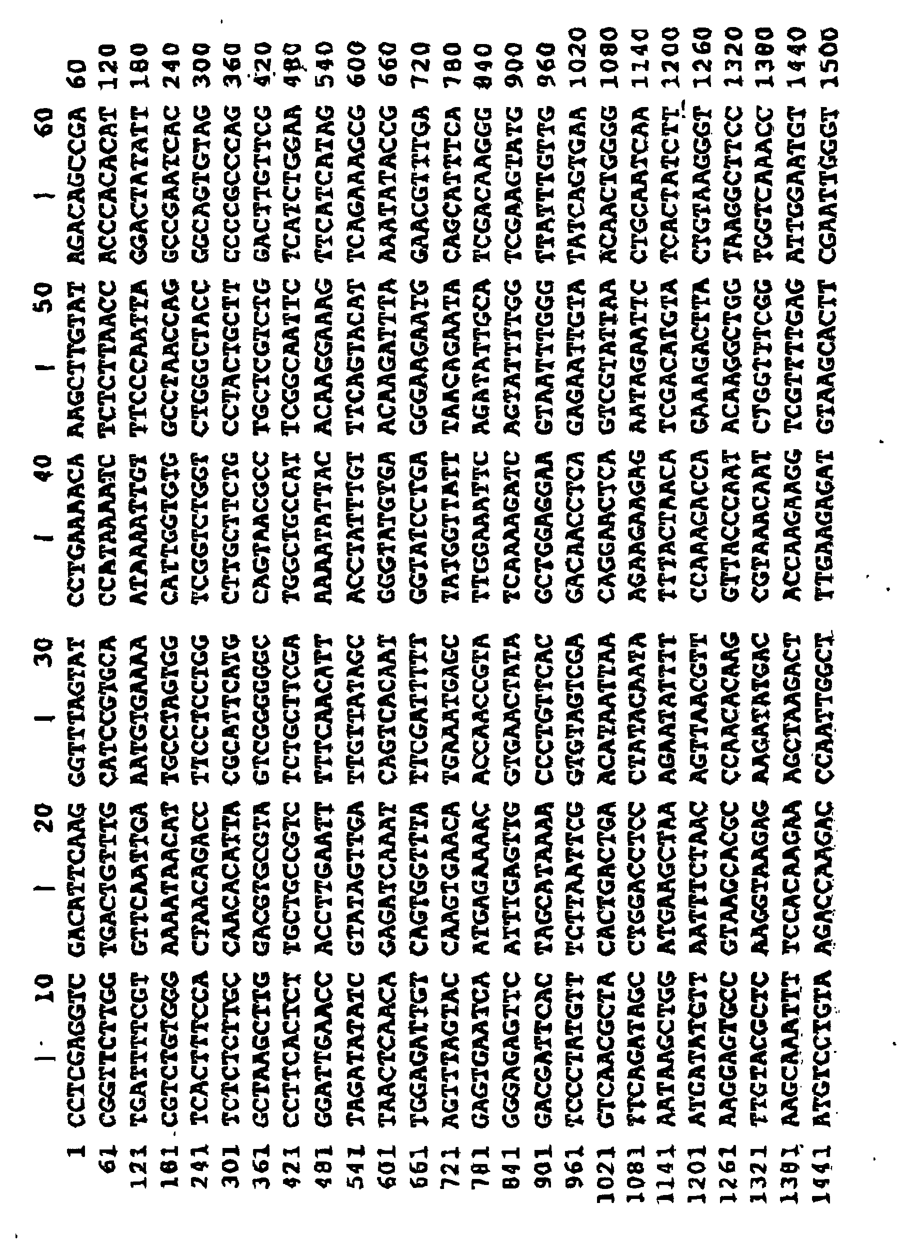 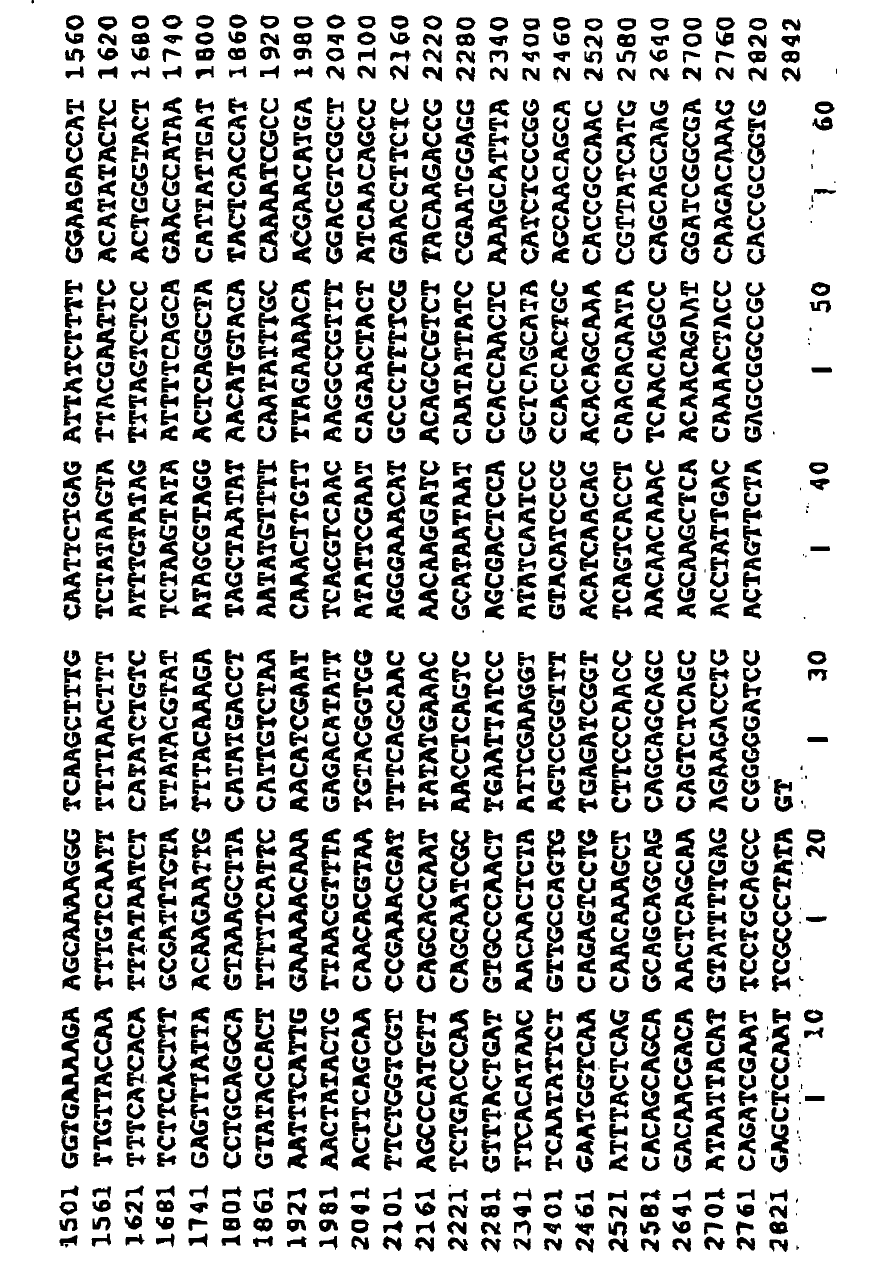 ou une séquence hybridant avec la séquence complémentaire de l'enchaînement II dans des conditions des forts stringence, et capable de conférer une résistance à une concentration de cycloheximide supérieure à 100 µg/ml, de préférence supérieure à 1 mg/ml, chez K. lactis.

5. Séquence nucléctidique selon l'une quelconque des revendications 1 à 4, **caractérisée en ce qu'**elle est obtenue à partir de la souche E.coli DH5α transformée par le plasmide Ye 23/31 déposée à la CNCM le 2 Juillet 1991, sous le numéro I-1121.

6. Séquence nuclèotidique **caractérisée en ce qu'**elle contient tout ou partie de la séquence II suivante : 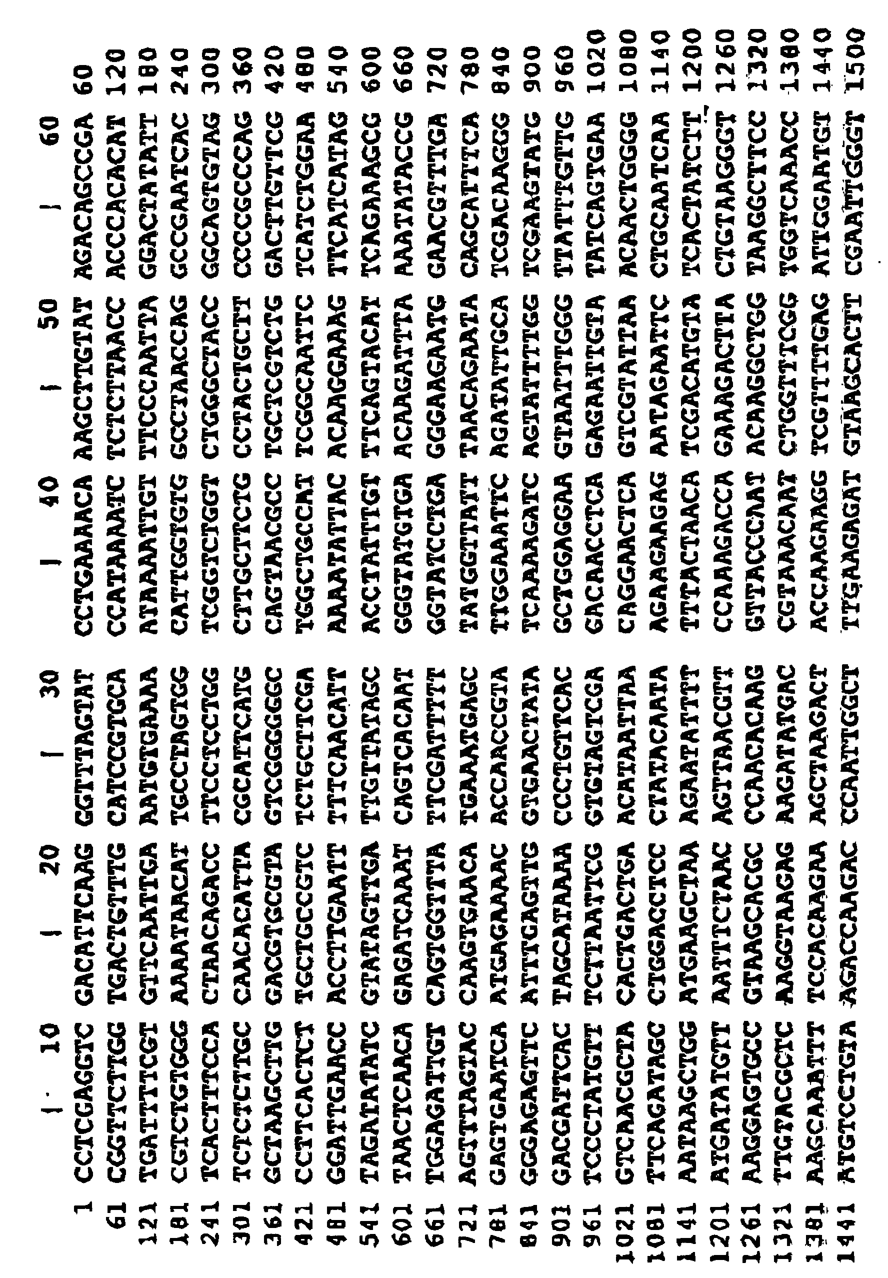 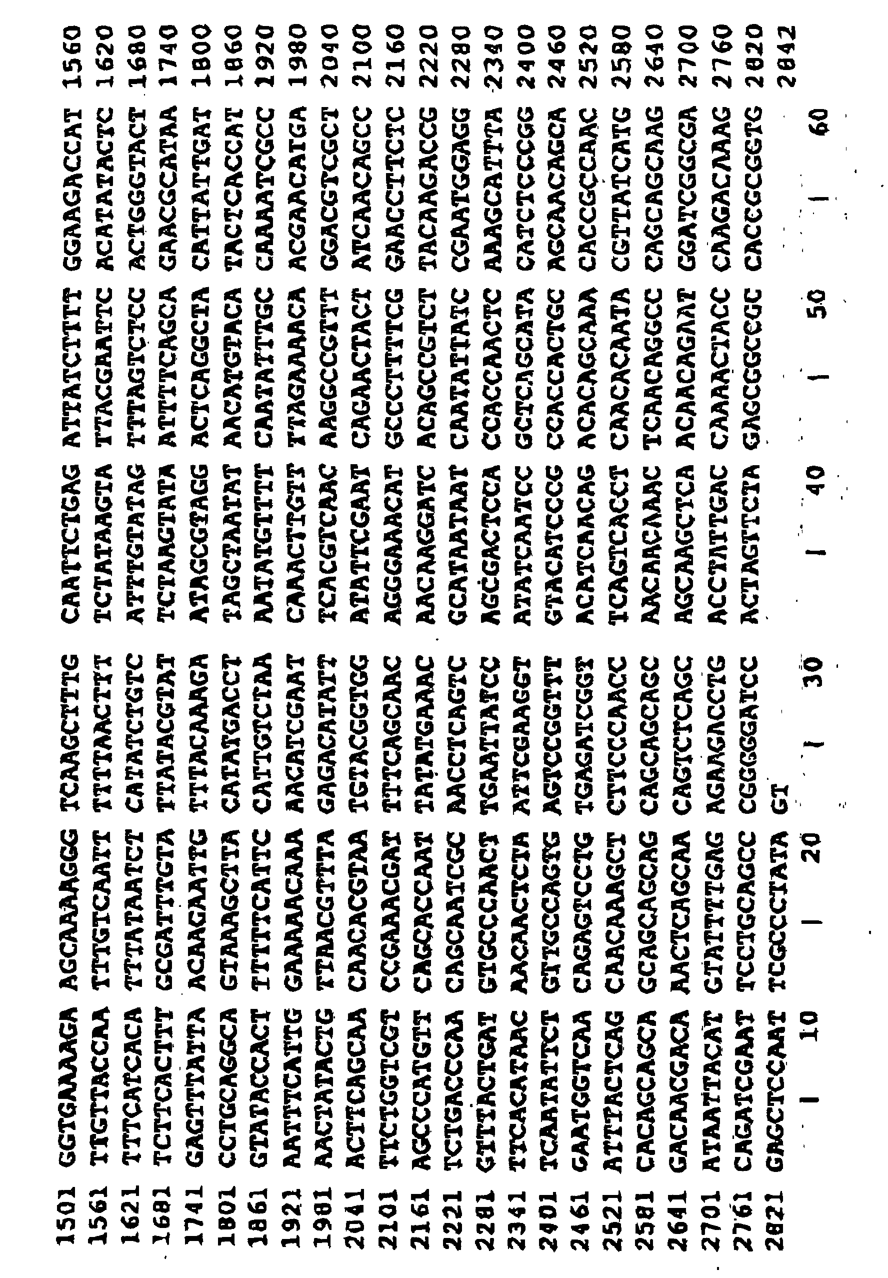 ou **en ce qu'**elle consiste en la séquence II ou **en ce qu'**elle hybride dans des conditions de forte stringence avec la séquence complémentaire de II dès lors que la séquence nucléotidique ainsi formée possède au moins l'une des propriétés suivantes :
- elle code pour une protéine de résistance à la cycloheximide et/ou,
- elle code pour un enchaînement d'acides aminés susceptibles d'être reconnu par des anticorps dirigés contre la protéine de résistance à la cycloheximide ayant la séquence A suivante : 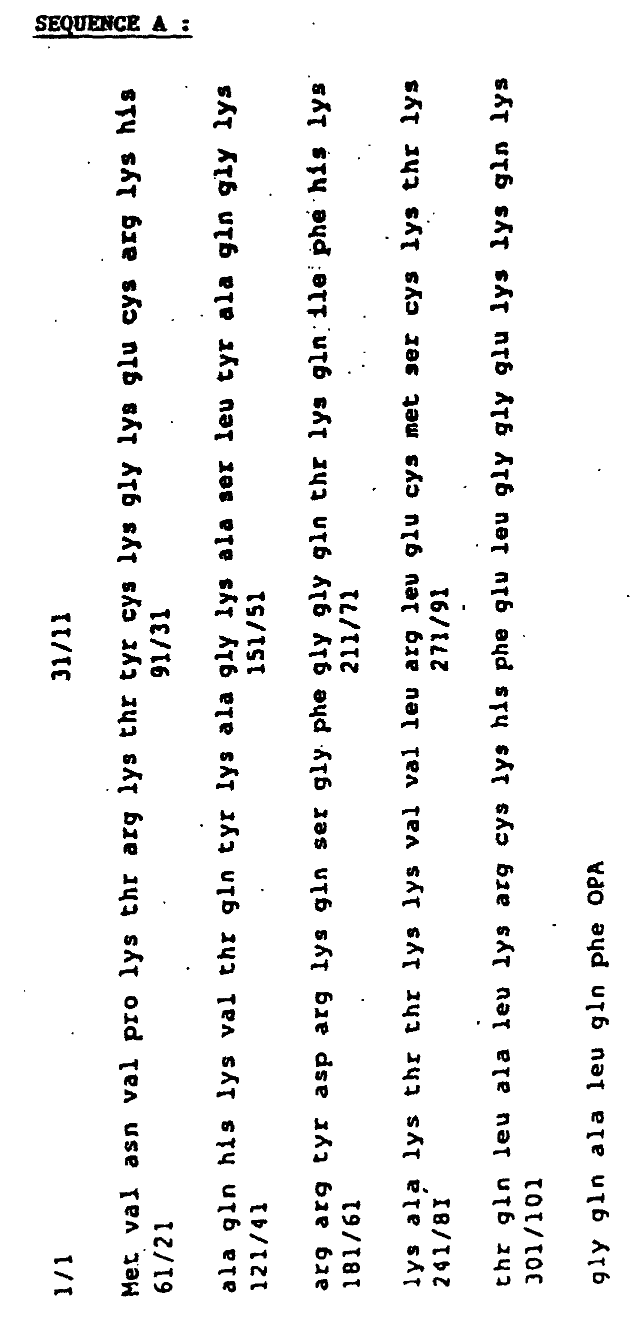 et/ou,
- elle régule l'expression de la séquence codant pour une protéine selon l'une quelconque des revendications 1 à 4 dans Kluyver omyces lactis.

7. Séquence nucléotidique selon la revendication 6, **caractérisée en ce qu'**elle contient l'enchaînement nucléotidique ORF A comprenant les nucléotidiques 1 à 1560 de l'enchaînement représenté à la figure 2, ou **en ce qu'**elle hybride avec la séquence complémentaire de ORF A dans des conditions de forte stringence.

8. Séquence nucléotidique selon la revendication 7, **caractérisée en ce qu'**elle contient la séquence codante I suivante de ORF A :

Séquence I

AT GGTTAACGTT CCAAAGACCA GAAAGACTTA CTGTAAGGGT
AAGGAGTGCC GTAAGCACGC CCAACACAAG GTTACCCAAT ACAAGGCTGG TAAGGCTTCC
TTGTACGCTC AAGGTAAGAG AAGATATGAC CGTAAACAAT CTGGTTTCGG TGGTCAAACC
AAGCAAATTT TCCACAAGAA AGCTAAGACT ACCAAGAAGG TCGTTTTGAG ATTGGAATGT
ATGTCCTGTA AGACCAAGAC CCAATTGGCT TTGAAGAGAT GTAAGCACTT CGAATTGGGT
GGTGAAAAGA AGCAAAAGGG TCAAGCTTTG CAATTCTGA

9. Séquence nucléotidique selon la revendication 6, **caractérisée en ce qu'**elle contient l'enchaînement nucléotidique III comprenant les nucléotides 1 à 628 de l'enchaînement représenté à la figure 2 ou la région promotrice de la transcription de la séquence ORF A incluse dans ledit enchaînement nucléotidique III.

10. Cofacteur susceptible d'augmenter la résistance à la cycloheximide conférée par une protéine codée par une séquence nucléotidique selon l'une quelconque des revendications 7 ou 8, **caractérisé en ce qu'**il est compris dans la séquence d'acide nucléique comprise entre les nucléotides 1561 à 2740 de l'enchaînement représenté à la figure 2 dès lors qu'il permet d'obtenir un niveau de résistance à la cycloheximide supérieur à 100 µg/ml chez un hôte eucaryote modifié par insertion de cette séquence d'acide nucléique et de la séquence nucléotidique codant pour une protéine de résistance, dans des conditions permettant la production de ces protéines.

11. Protéine de résistance à la cycloheximide, **caractérisée en ce qu'**elle consiste en l'enchaînement d'acides aminés A suivante : 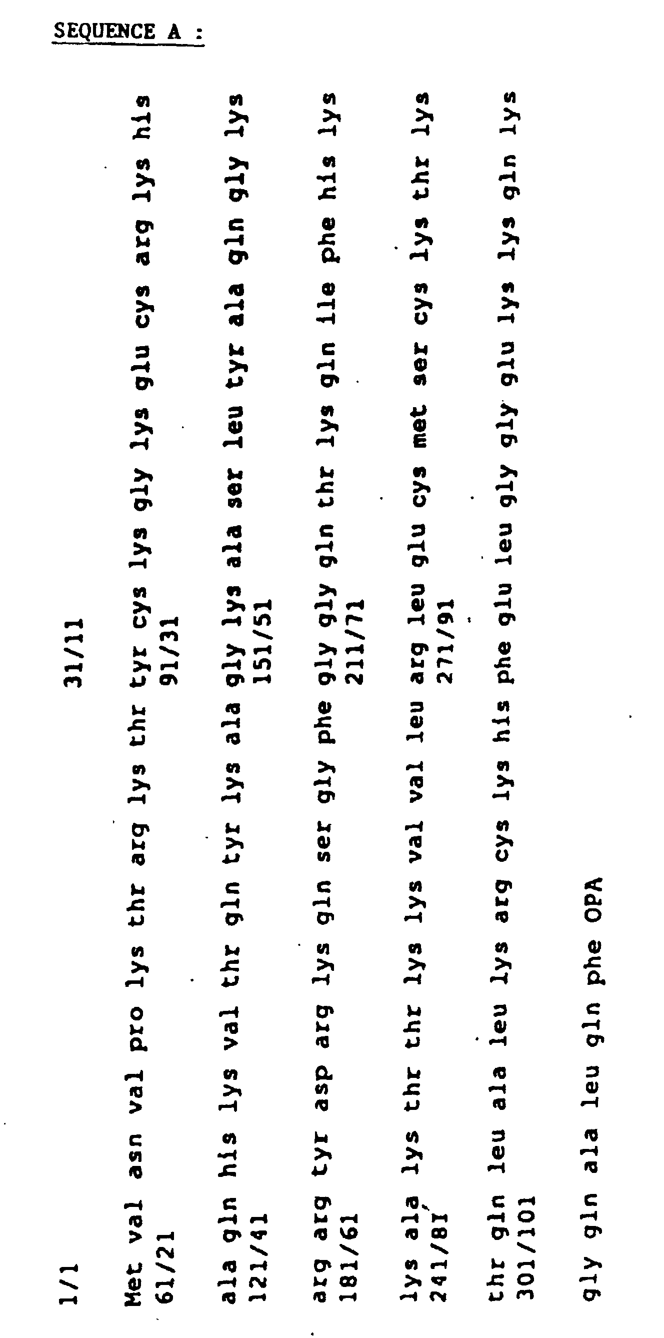 ou **en ce qu'**elle comprend tout ou partie de cet enchaînement A éventuellement modifié, dès lors que la protéine formée confère une résistance à une concentration supérieure ou égale à 100 µg/ml à la cycloheximide à un hôte eucaryote recombinant transformé par la séquence nucléotidique codant pour cette protéine, dans des conditions permettant sa production.

12. Protéine de résistance à la cycloheximide, **caractérisée en ce qu'**elle est codée par la séquence nucléotidique I suivante : 
Séquence I

AT GGTTAACGTT CCAAAGACCA GAAAGACTTA CTGTAAGGGT
AAGGAGTGCC GTAAGCACGC CCAACACAAG GTTACCCAAT ACAAGGCTGG TAAGGCTTCC
TTGTACGCTC AAGGTAAGAG AAGATATGAC CGTAAACAAT CTGGTTTCGG TGGTCAAACC
AAGCAAATTT TCCACAAGAA AGCTAAGACT ACCAAGAAGG TCGTTTTGAG ATTGGAATGT
ATGTCCTGTA AGACCAAGAC CCAATTGGCT TTGAAGAGAT GTAAGCACTT CGAATTGGGT
GGTGAAAAGA AGCAAAAGGG TCAAGCTTTG CAATTCTGA
 par une partie de la séquence I ou par une séquence modifiée par rapport à I, dès lors que la protéine codée par cette partie de séquence ou cette séquence modifiée est capable de conférer une résistance à une concentration supérieure ou égale à 100 µg/ml à la cycloheximide lorsqu'elle est introduite chez un hôte eucaryote, dans des conditions permettant son expression.

13. Protéine codée par une séquence d'acide nucléique selon l'une quelconque des revendications 1 à 10, telle qu'obtenue à partir de Kluyveromyces lactis.

14. Vecteur recombinant, en particulier pour le clonage et/ou l'expression, notamment du type plasmide, **caractérisé en ce qu'**il comprend au moins une séquence nucléotidique selon l'une quelconque des revendications 1 à 10, sous le contrôle des éléments de régulation nécessaires à son expression dans un hôte eucaryote choisi, ces éléments de régulation comportant un promoteur, le cas échéant inductible et une séquence de terminaison de transcription.

15. Vecteur selon la revendication 14, **caractérisé en ce qu'**il s'agit d'un vecteur adapté à l'expression dans les levures telles que Pichia Pastoris, ou d'un vecteur adapté à l'expression dans une cellule animale, par exemple le baculovirus, ou d'un vecteur adapté à l'expression dans une cellule végétale.

16. Vecteur selon l'une quelconque des revendications 14 ou 15, **caractérisé en ce qu'**il contient en outre un acide nucléique déterminé dont on recherche l'expression dans un hôte eucaryote choisi, cet acide nucléique étant sous le contrôle d'éléments de régulation nécessaires à son expression dans l'hôte.

17. Vecteur selon la revendication 16, **caractérisé en ce que** l'acide nucléique est sous le contrôle des éléments de régulation contrôlant la transcription des séquences nucléotidiques selon l'une quelconque des revendications 1 à 10.

18. Cellule eucaryote **caractérisée en ce qu'**elle est transformée par un vecteur selon l'une quelconque des revendications 14 à 17 et en particulier **en ce qu'**il s'agit d'une cellule de levure, par exemple Pichia pastoris, d'une cellule animale, par exemple d'une cellule d'insecte ou d'une cellule de mammifère notamment murine ou simienne, d'une cellule humaine ou encore d'une cellule végétale.

19. Vecteur recombinant selon la revendication 14, **caractérisé en ce qu'**il est contenu dans la souche E. coli DH5α déposée à la CNCM le 2 Juillet 1991, sous le numéro I-1121.

20. Utilisation d'un vecteur selon l'une quelconque des revendications 14 à 17, dans un système de sélection répondant spécifiquement à un marqueur de type cycloheximide, chez un hôte eucaryote, notamment pour contrôler chez cet hôte l'incorporation d'un acide nucléique hétérologue déterminé.

21. Procédé pour le contrôle de la présence d'un acide nucléique hétérologue dans un hôte cellulaire, **caractérisé en ce qu'**il comprend :
- la transformation de l'hôte cellulaire par un vecteur d'expression comportant en des sites non essentiels pour sa réplication, l'acide nucléique hétérologue, une séquence nucléotidique selon l'une quelconque des revendications 1 à 10, sous le contrôle des éléments de régulation nécessaires à l'expression de ces séquences dans l'hôte cellulaire choisi,
- la culture de l'hôte cellulaire ainsi transformé,
- la mise en contact de l'hôte avec une concentration déterminée de cycloheximide et la détection de la résistance de l'hôte vis à vis de cet antibiotique.

## Claims

1. A nucleic acid sequence of about 5.2 kb from K. lactis, capable of conferring resistance to cycloheximide in a concentration of more than 1 mg/ml in K. lactis or in S. cerevisiae and comprising the restriction sites: Sau3A: 0, StuI: +300 bp, PvuII: +600 bp, BgIII: +900 bp BstXI: +1200 bp, PvuII: +2300 bp, PstI: +2400 bp, PstI: +3000 bp, EcoRI: +3200 bp, HindIII: +3300 bp, EcoRI: +3700 bp, HindIII: +4500 bp, HindIII: +4540 bp, SalI: +5100 bp.

2. A nucleic acid sequence of about 5.2 kb according to claim 1, comprising the following nucleotide concatenation I: 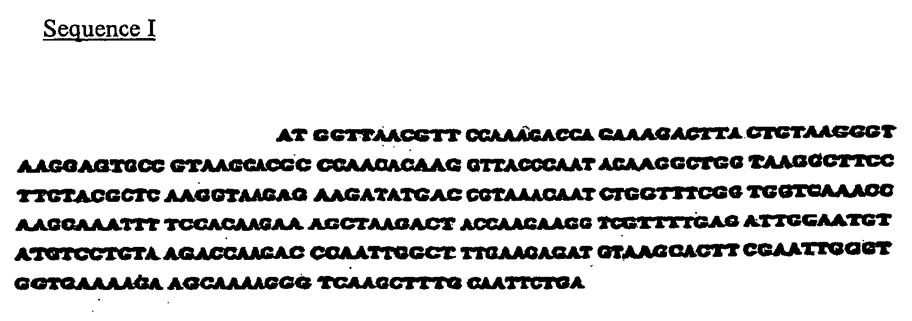 or hybridizing with the sequence complementary to concatenation I, under high stringent conditions (0.1 x SSC, 0.1% SDS at 65°C), and capable of conferring resistance to cycloheximide in a concentration of more than 100 µg/ml, preferably more than 1 mg/ml, in K. lactis.

3. A nucleic acid sequence of about 5.2 kb according to claim 1 or claim 2, comprising the following nucleotide concatenation II: 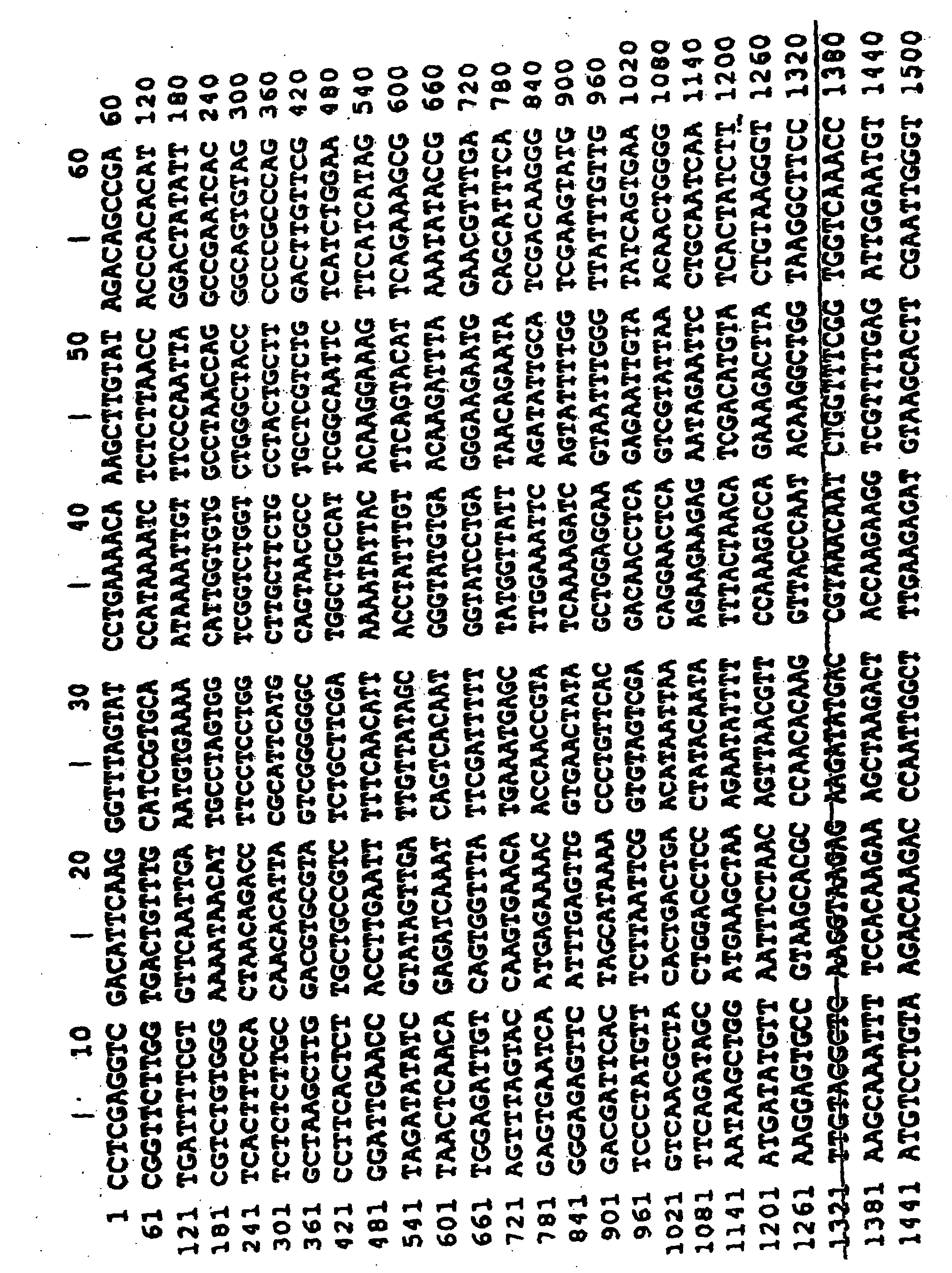 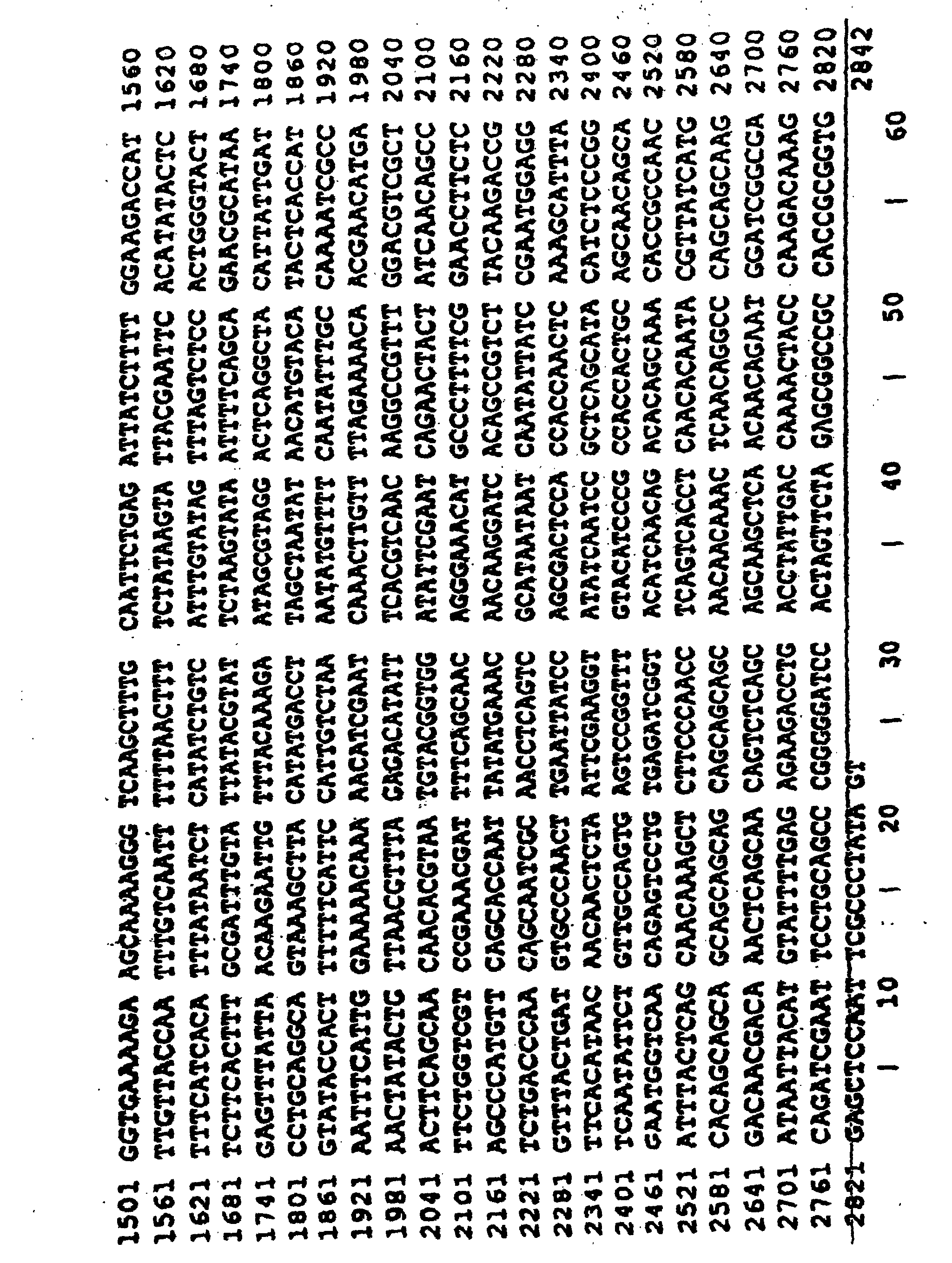 or hybridizing with the sequence complementary to concatenation II under high stringent conditions, and capable of conferring resistance to a cycloheximide concentration of more than 100 µg/ml, preferably more than 1 mg/ml, in K. lactis.

4. A nucleic acid sequence according to claim 1 or claim 2, comprising the sequence of nucleotides located between positions 9 and 2763 of the following concatenation II: 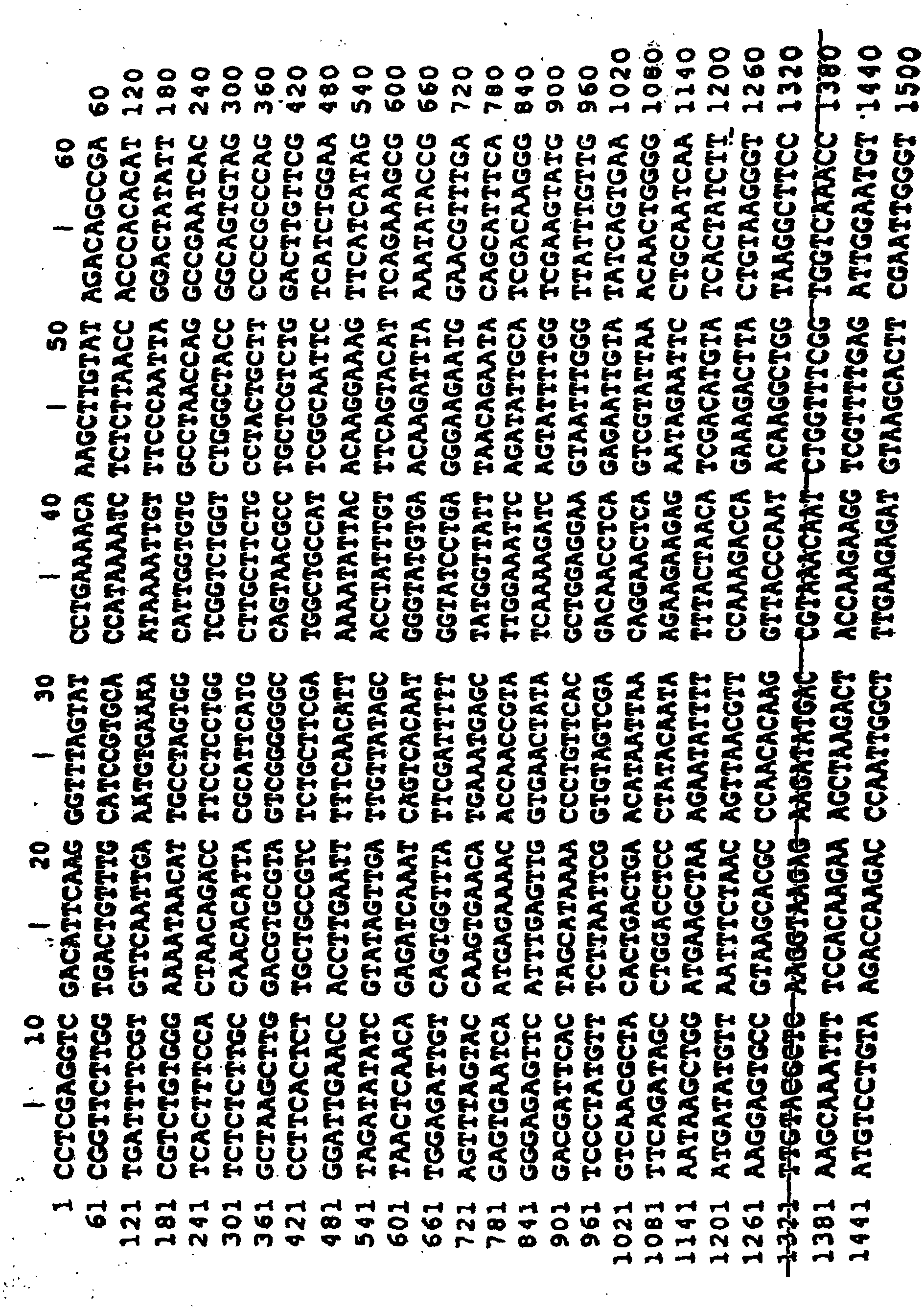 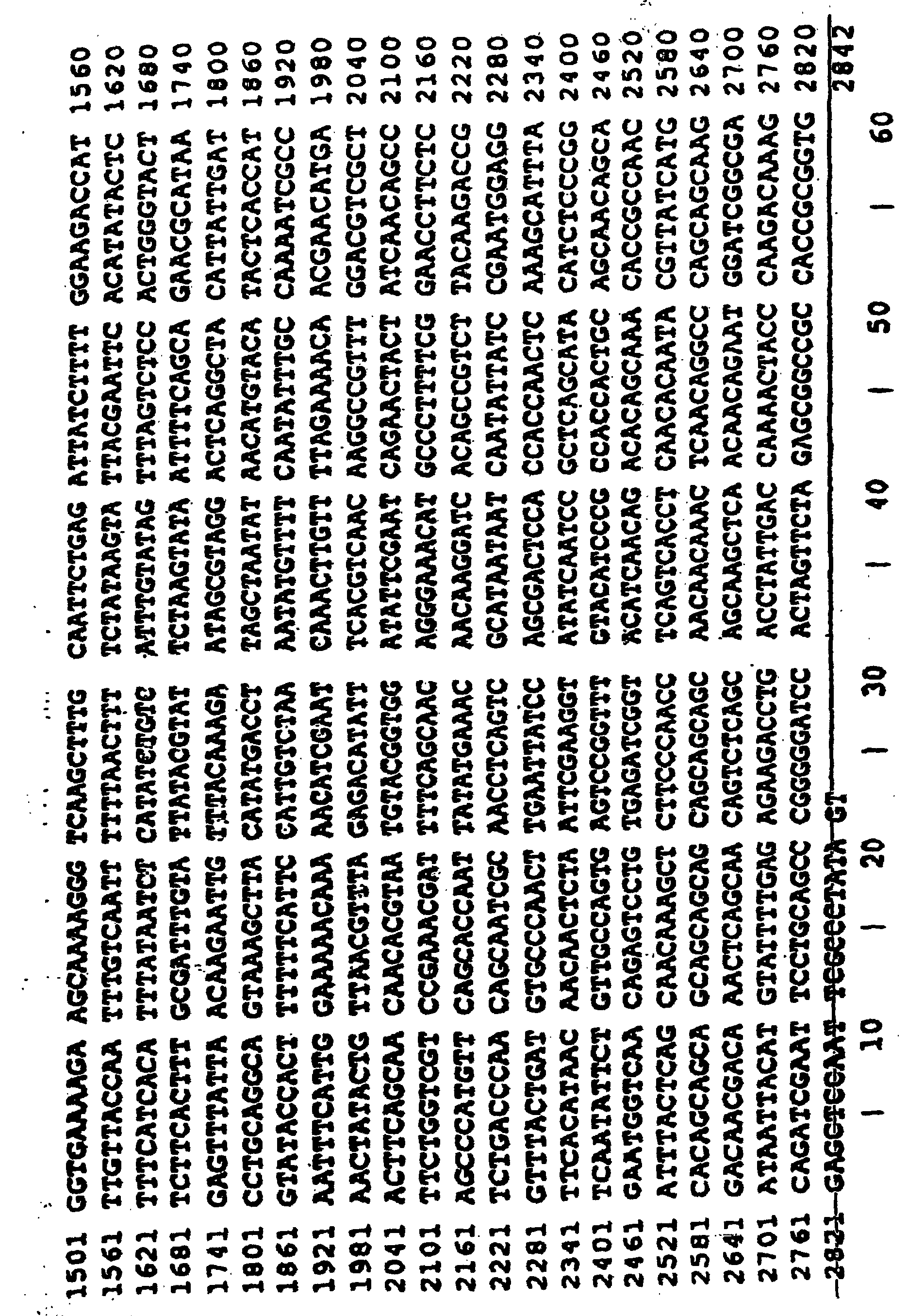 or a sequence hybridizing with the sequence complementary to concatenation II under high stringent conditions, and capable of conferring resistance to a cycloheximide concentration of more than 100µg/ml, preferably more than 1 mg/ml, in K. lactis.

5. A nucleotide sequence according to any one of claims 1 to 4, **characterized in that** it is obtained from the E. coli DH5α strain transformed by the plasmid Ye 23/31 deposited with the CNCM on 2nd July 1991, with accession number I-1121.

6. A nucleotide sequence, **characterized in that** it contains all or a part of the following sequence II: 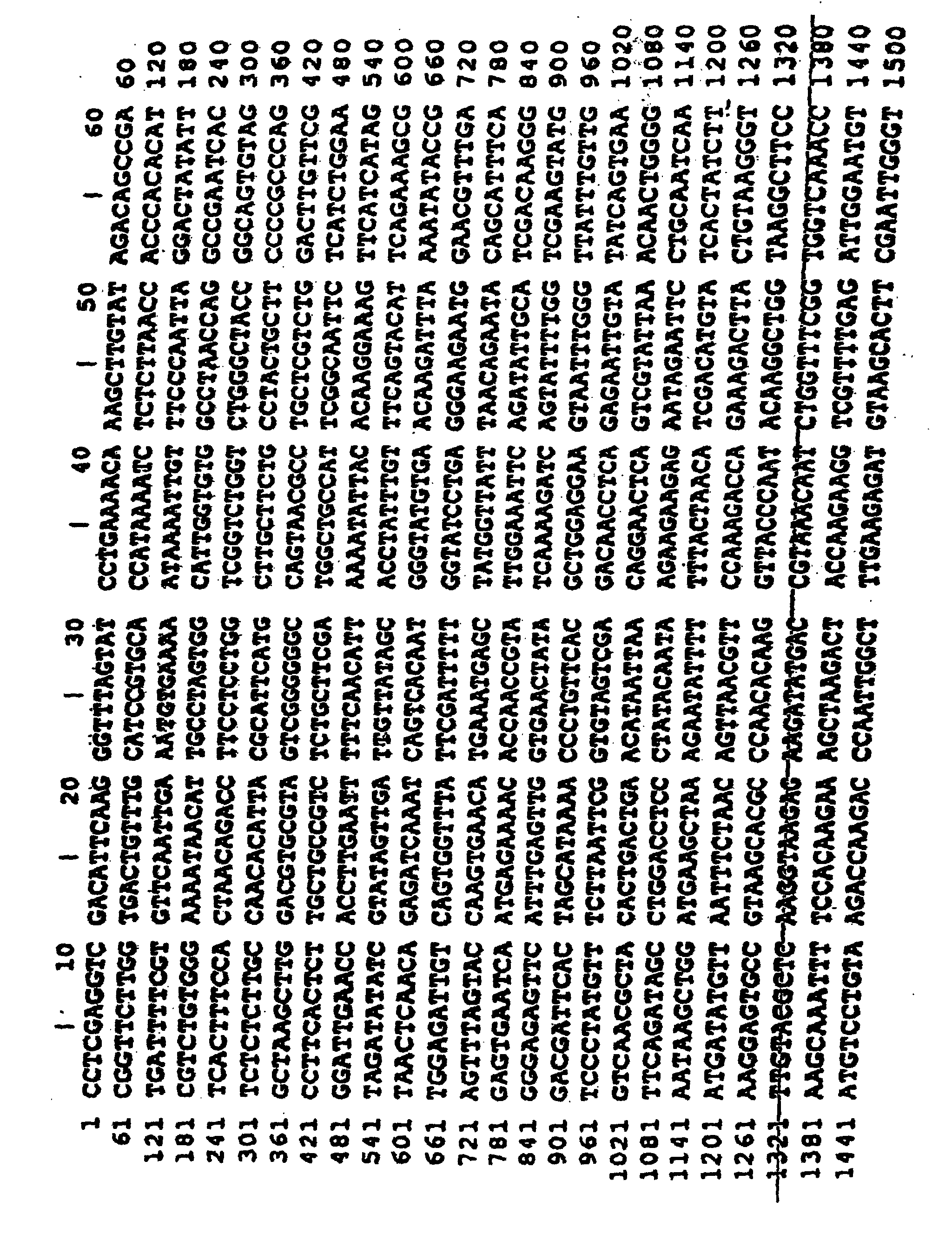 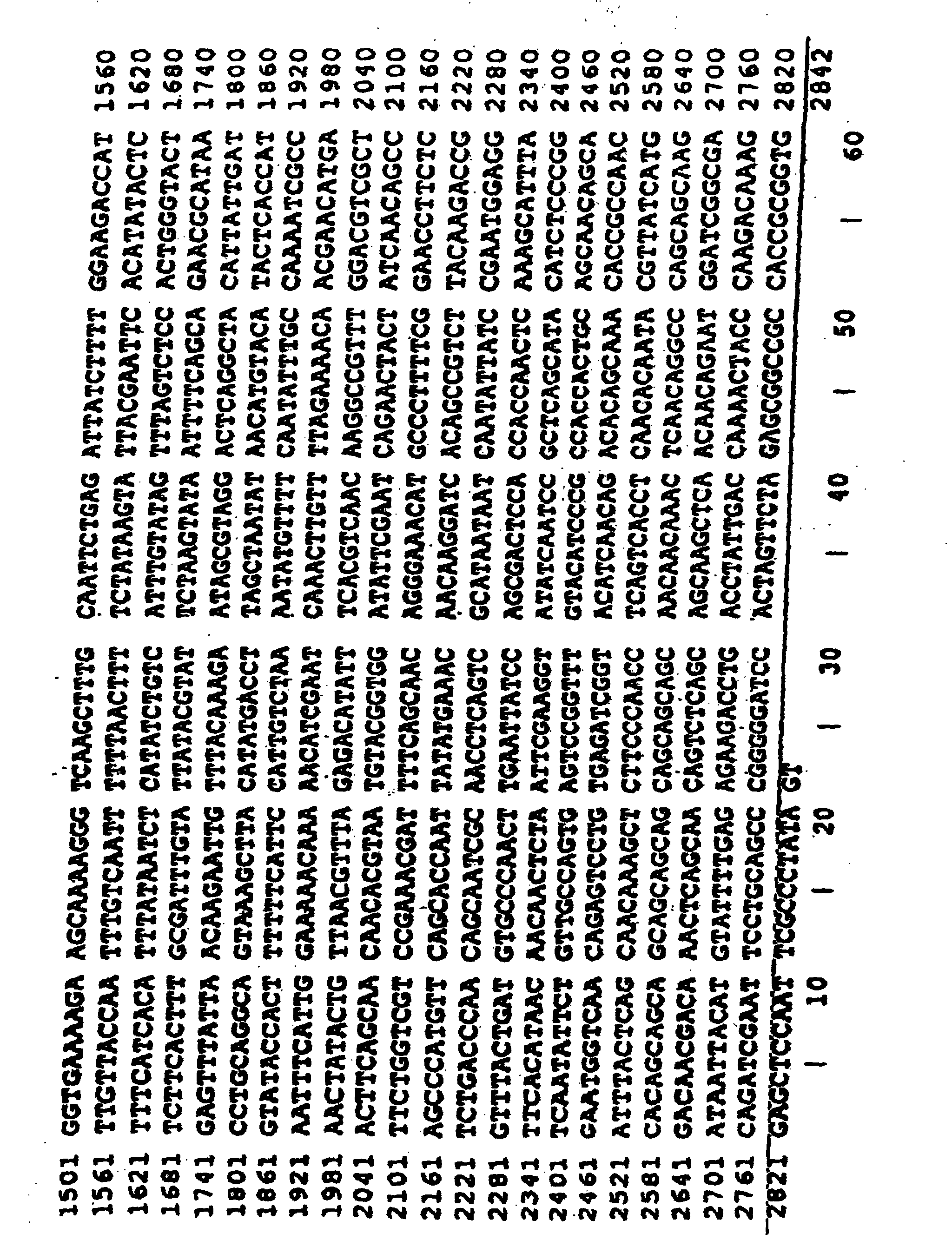 or **in that** it consists of sequence II or **in that** it hybridizes under high stringent conditions with the complementary sequence of II provided that the nucleotide sequence formed possesses at least one of the following properties:
• it codes for a protein for cycloheximide resistance; and/or
• it codes for a concatenation of amino acids capable of being recognized by antibodies directed against the protein for cycloheximide resistance having the following sequence A: 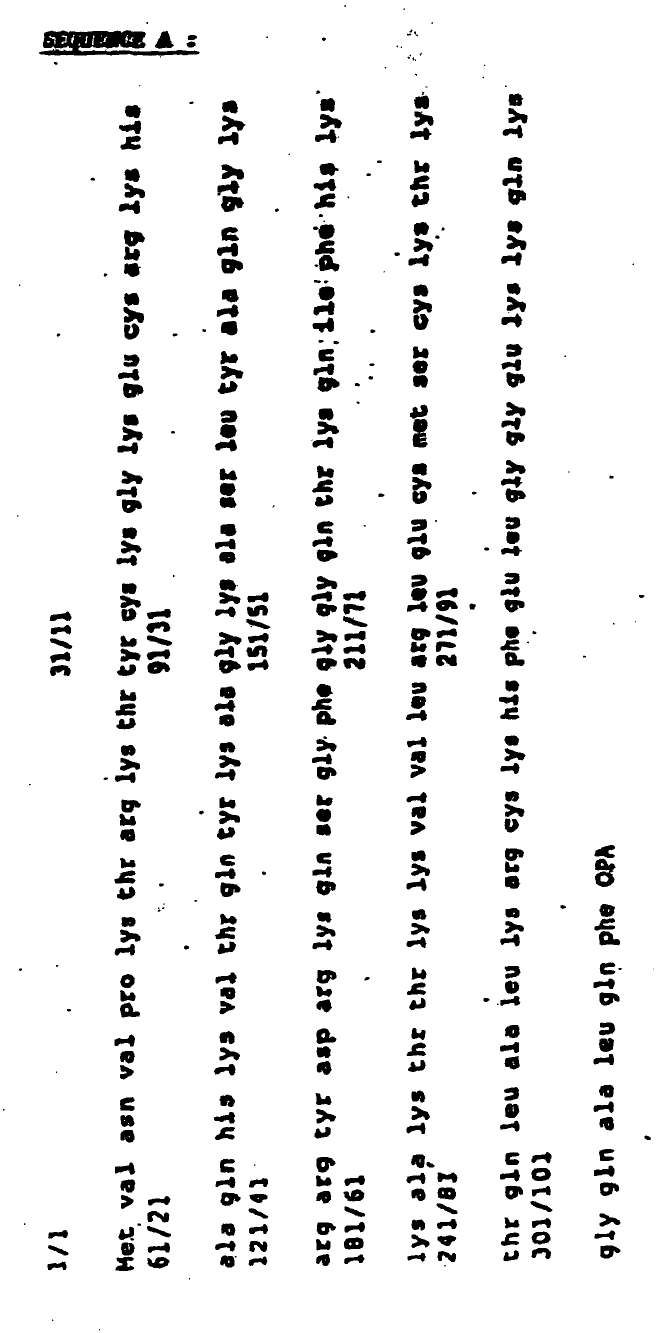 and/or
• it regulates expression of the sequence coding for a protein according to any one of claims 1 to 4 in Kluyveromyces lactis.

7. A nucleotide sequence according to claim 6, **characterized in that** it contains the nucleotide concatenation ORF A comprising nucleotides 1 to 1560 of the concatenation shown in Figure 2, or **in that** it hybridizes with the sequence complementary to ORF A under high stringent conditions.

8. A nucleotide sequence according to claim 7, **characterized in that** it contains the following coding sequence I of ORF A:

Sequence I

AT GGTTAACGTT CCAAAGACCA GAAAGACTTA CTGTAAGGGT
AAGGAGTGCC GTAAGCACGC CCAACACAAG GTTACCCAAT ACAAGGCTGG TAAGGCTTCC
TTGTACCCTC AAGGTAAGAG AAGATATGAC CGTAAACAAT CTGGTTTCGG TGGTCAAACC
AAGCAAATTT TCCACAAGAA AGCTAAGACT ACCAAGAAGG TCGTTTTGAG ATTGGAATGT
ATGTCCTGTA AGACCAAGAC CCAATTGGCT TTGAAGAGAT GTAAGCACTT CGAATTGGGT
GGTGAAAAGA AGCAAAAGGG TCAAGCTTTG CAATTCTGA

9. A nucleotide sequence according to claim 6, **characterized in that** it contains the nucleotide concatenation III comprising nucleotides 1 to 628 of the concatenation shown in Figure 2 or the region for promoting transcription of the sequence ORF A included in said nucleotide concatenation III.

10. A co-factor capable of enhancing resistance to cycloheximide conferred by a protein coded by a nucleotide sequence according to claim 7 or claim 8, **characterized in that** it is included in the nucleic acid sequence included between nucleotides 1561 to 2740 of the concatenation shown in Figure 2 provided that it can produce a level of resistance at a cycloheximide concentration of more than 100µg/ml in a eukaryotic host modified by inserting said nucleic acid sequence and the nucleotide sequence coding for a resistance protein, under conditions allowing the production of said proteins.

11. A protein for resistance to cycloheximide, **characterized in that** it consists of the following concatenation of amino acids A: 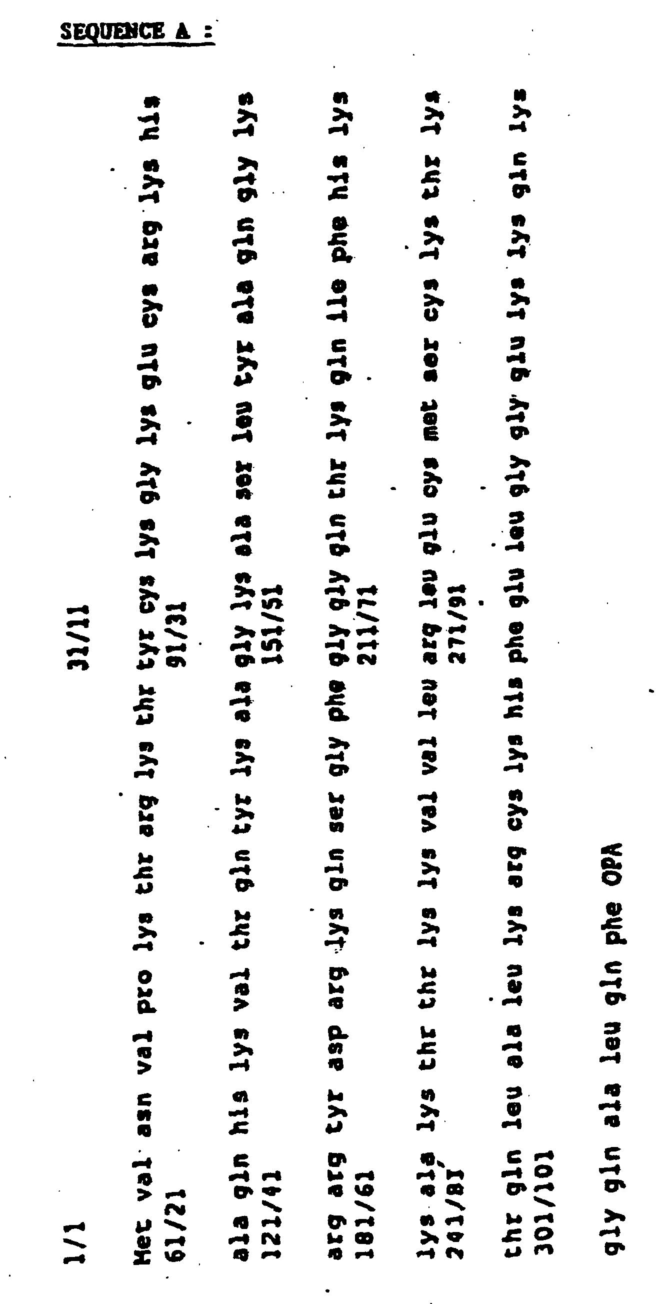 or **in that** it comprises all or a part of said concatenation A, possibly be modified, provided that the protein formed confers resistance at a cycloheximide concentration of 100 µg/ml or more in a recombinant host eukaryote transformed by the nucleotide sequence coding for this protein, under conditions allowing its production.

12. A protein for cycloheximide resistance, **characterized in that** it is coded by the following nucleotide sequence I: 
Sequence I:

AT GCTTAACGTT CCAAAGACCA GAAAGACTTA CTGTAAGGGT
AAGGAGTGCC GTAAGCACGC CCAACACAAG GTTACCCAAT ACAAGGCTGG TAAGGCTTCC
TTGTACGCTC AAGGTAAGAG AAGATATGAC CGTAAACAAT CTGGTTTCGG TCGTCAAACC
AAGCAAATTT TCCACAAGAA AGCTAAGACT ACCAAGAAGG TCGTTTTGAG ATTGGAATCT
ATGTCCTGTA AGACCAAGAC CCAATTGGGT TTGAAGAGAT GTAAGCACTT CGAATTGGGT
GGTGAAAAGA AGCAAAAGGG TCAAGCTTTG CAATTGTGA
 by a part of the sequence I or by a sequence modified with respect to I, provided that the protein coded by said part of sequence or said modified sequence is capable of conferring resistance at a cycloheximide concentration of 100 µg/ml or more when it is introduced into a eukaryotic host, under conditions allowing its expression.

13. A protein coding for a nucleic acid sequence according to any one of claims 1 to 10, obtained from Kluyveromyces lactis.

14. A recombinant vector, in particular for cloning and/or expression, in particular of the plasmid type, **characterized in that** it comprises at least one nucleotide sequence according to any one of claims 1 to 10, under the control of the regulating elements necessary for its expression in a selected eukaryotic host, said regulating elements comprising a promoter, which may be inducible, and a transcription termination sequence.

15. A vector according to claim 14, **characterized in that** said vector is adapted for expression in yeasts such as Pichia pastoris, or an adapted vector for expression in an animal cell, for example baculovirus, or an adapted vector for expression in a plant cell.

16. A vector according to any one of claims 14 or 15, **characterized in that** it further contains a predetermined nucleic acid the expression of which is sought in a given eukaryotic host, said nucleic acid being under the control of regulation elements necessary to its expression in the host.

17. A vector according to claim 16, **characterized in that** the nucleic acid is under the control of regulating elements controlling the transcription of nucleotide sequences according to any one of claims 1 to 10.

18. A eukaryotic cell, **characterized in that** it is transformed by a vector according to any one of claims 14 to 17 and in particular **in that** it is a yeast cell, for example Pichia pastoris, an animal cell, for example an insect cell or a mammalian cell, in particular murine or simian, a human cell or a plant cell.

19. A recombinant vector according to claim 14, **characterized in that** it is contained in the E. coli DH5α strain deposited at the CNCM on 2nd July 1991, with accession number I-1121.

20. Use of a vector according to any one of claims 14 to 17, in a selection system responding specifically to a cycloheximide type marker in a eukaryotic host, in particular to monitor the incorporation of a predetermined heterologous nucleic acid in said host.

21. A process for monitoring the presence of a heterologous nucleic acid in a host cell, **characterized in that** it comprises:
• transforming the host cell by an expression vector comprising, at non essential sites to its replication, the heterologous nucleic acid, a nucleotide sequence according to any one of claims 1 to 10, under the control of the regulating elements necessary for expression of said sequences in the selected host cell;
• culturing the transformed host cell;
• bringing the host into contact with a predetermined concentration of cycloheximide and detecting the resistance of the host to said antibiotic.

## Patentansprüche

1. Nukleinsäuresequenz von K. lactis von etwa 5,2 kb, die fähig ist, bei K. lactis oder bei S. cerevisiae eine Resistenz gegen Cycloheximid in einer Konzentration höher als 1 mg/ml zu verleihen und die Restriktionsstellen umfasst: Sau3A : 0, StuI : +300 bp, PvuII : +600 bp, BglII : +900 bp, BstXI : +1200 bp, PvuII : +2300 bp, Pst I : +2400 bp, PstI : +3000 bp, EcoRI : +3200 bp, HindIII : +3300 bp, EcoRI : +3700 bp, HindIII : +4500 bp, HindIII : +4540 bp, SalI : +5100 bp.

2. Nukleinsäuresequenz von etwa 5,2 kb nach Anspruch 1, die die folgende Nukleotidverknüpfung I umfasst: 
Sequenz I

AT GGTTAACGTT CCAAAGACCA GAAAGACTTA CTGTAAGGGT
AAGGAGTGCC GTAAGCACGC CCAACACAAG GTTACCCAAT ACAAGGCTGG TAAGGCTTCC
TTGTACGCTC AAGGTAAGAG AAGATATGAC CGTAAACAAT CTGGTTTCGG TGGTCAAACC
AAGCAAATTT TCCACAAGAA AGCTAAGACT ACCAAGAAGG TCGTTTTGAG ATTGGAATGT
ATGTCCTGTA AGACCAAGAC CCAATTGGCT TTGAAGAGAT GTAAGCACTT CGAATTGGGT
GGTGAAAAGA AGCAAAAGGG TCAAGCTTTG CAATTCTGA
 oder mit der komplementären Sequenz der Verknüpfung I unter Bedingungen hoher Stringenz (0,1 x SSC, 0,1% SDS bei 65 °C) hybridisiert, und fähig ist, bei K. lactis eine Resistenz gegen Cycloheximid in einer Konzentration höher als 100 µg/ml zu verleihen, vorzugsweise höher als 1 mg/ml.

3. Nukleinsäuresequenz von etwa 5,2 kb nach Anspruch 1 oder 2, die die folgende Nukleotidverknüpfung II umfasst: 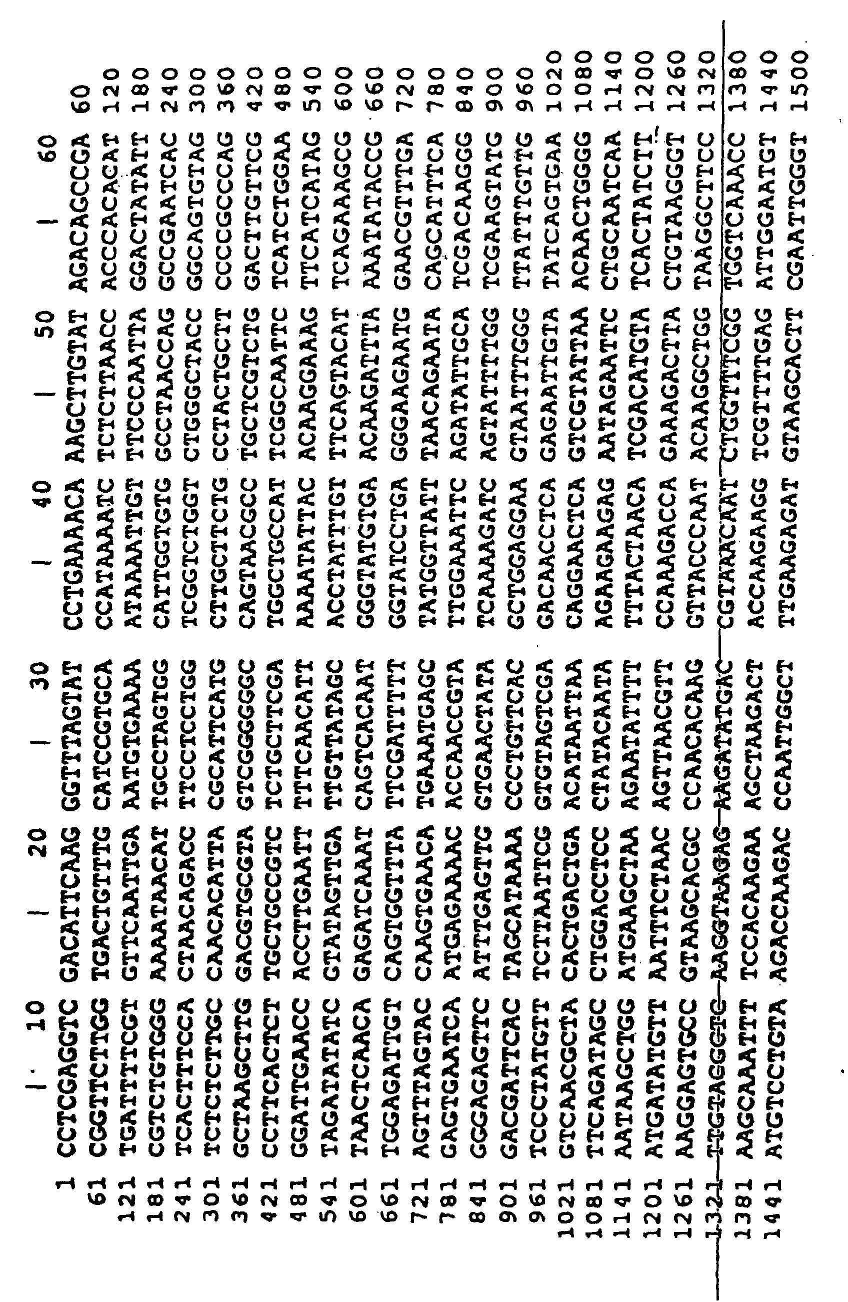 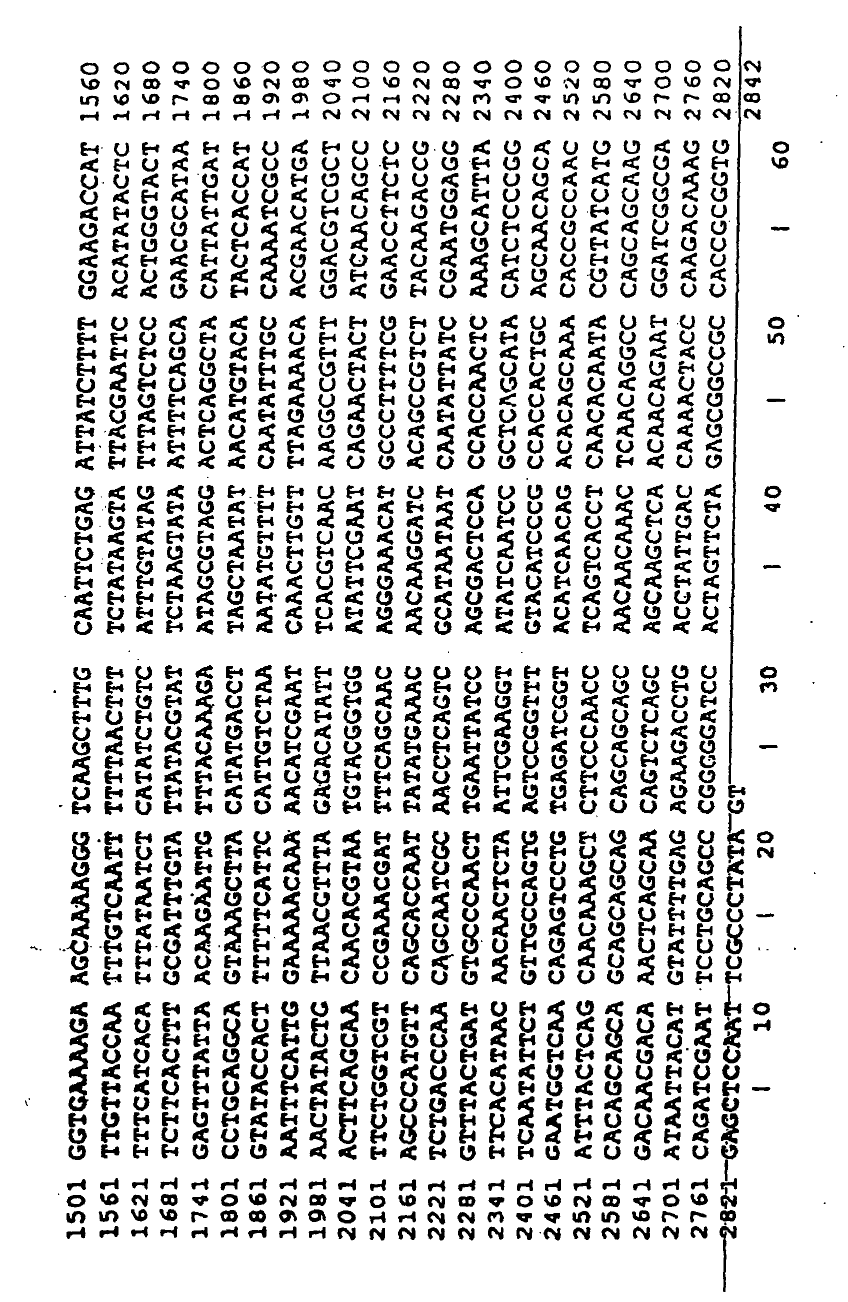 oder mit der komplementären Sequenz der Verknüpfung II unter Bedingungen hoher Stringenz hybridisiert und fähig ist, bei K. lactis eine Resistenz gegen eine Konzentration von Cycloheximid höher als 100 µg/ml, vorzugsweise höher als 1 mg/ml, zu verleihen.

4. Nukleinsäuresequenz nach Anspruch 1 oder 2, die die Nukleotidsequenz umfasst, die zwischen den Positionen 9 und 2763 der folgenden Verknüpfung II liegt: 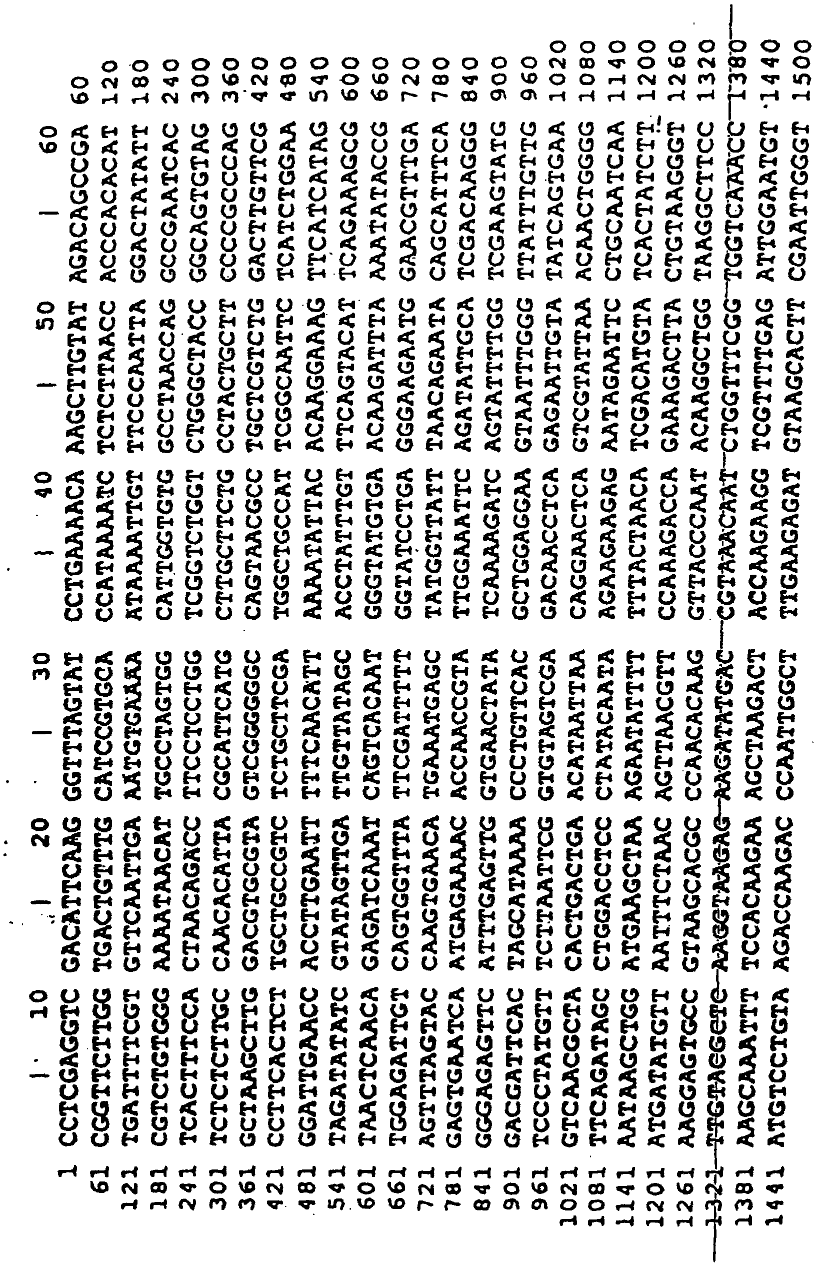 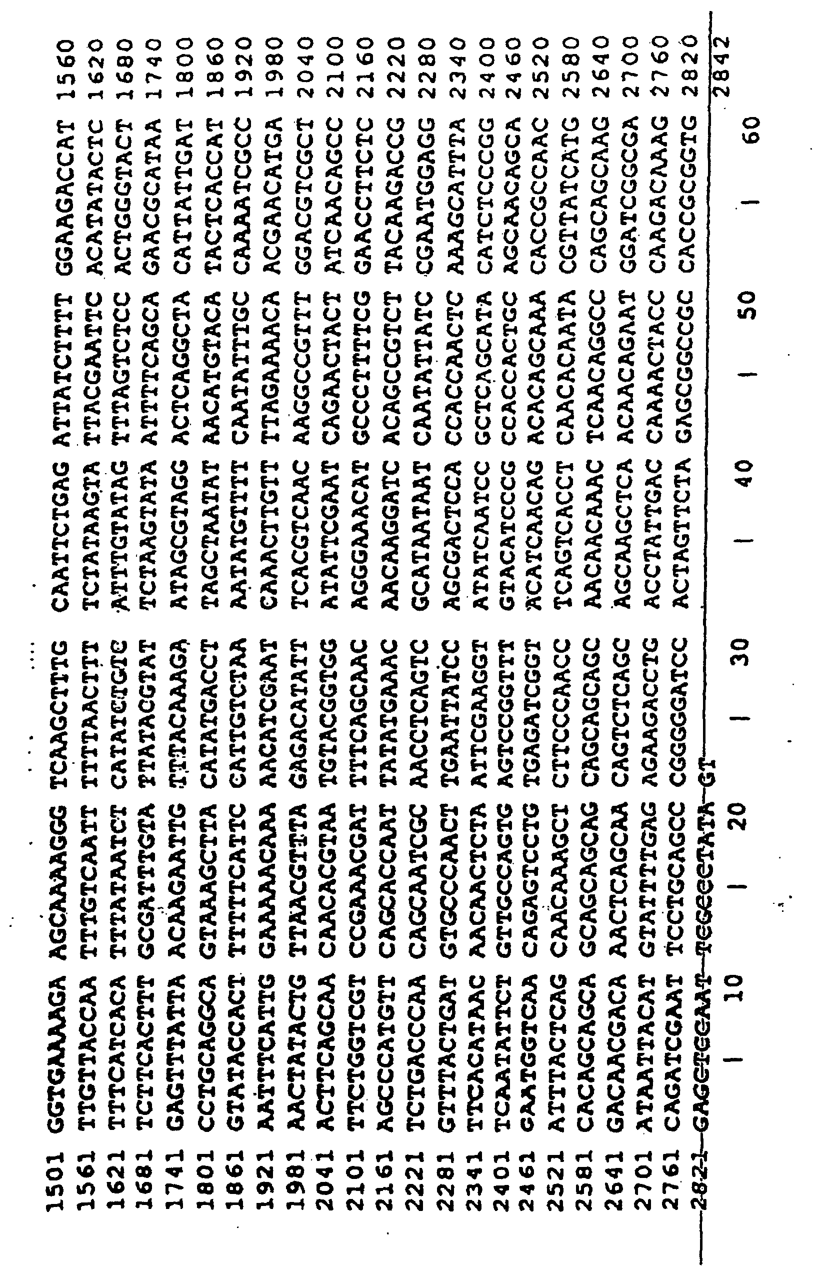 oder eine Sequenz, die mit der komplementären Sequenz der Verknüpfung II unter Bedingungen hoher Stringenz hybridisiert und fähig ist, bei K. lactis eine Resistenz gegen eine Konzentration von Cycloheximid höher als 100 µg/ml, vorzugsweise höher als 1 mg/ml, zu verleihen.

5. Nukleotidsequenz nach irgendeinem der Ansprüche 1 bis 4, **dadurch gekennzeichnet, dass** sie ausgehend von dem Stamm E. coli DH5α erhalten ist, der mit dem Plasmid Ye 23/31 transformiert wurde, das bei der CNCM am 2. Juli 1991 unter der Nummer I-1121 hinterlegt wurde.

6. Nukleotidsequenz, **dadurch gekennzeichnet, dass** sie die ganze oder einen Teil der folgenden Sequenz II enthält: 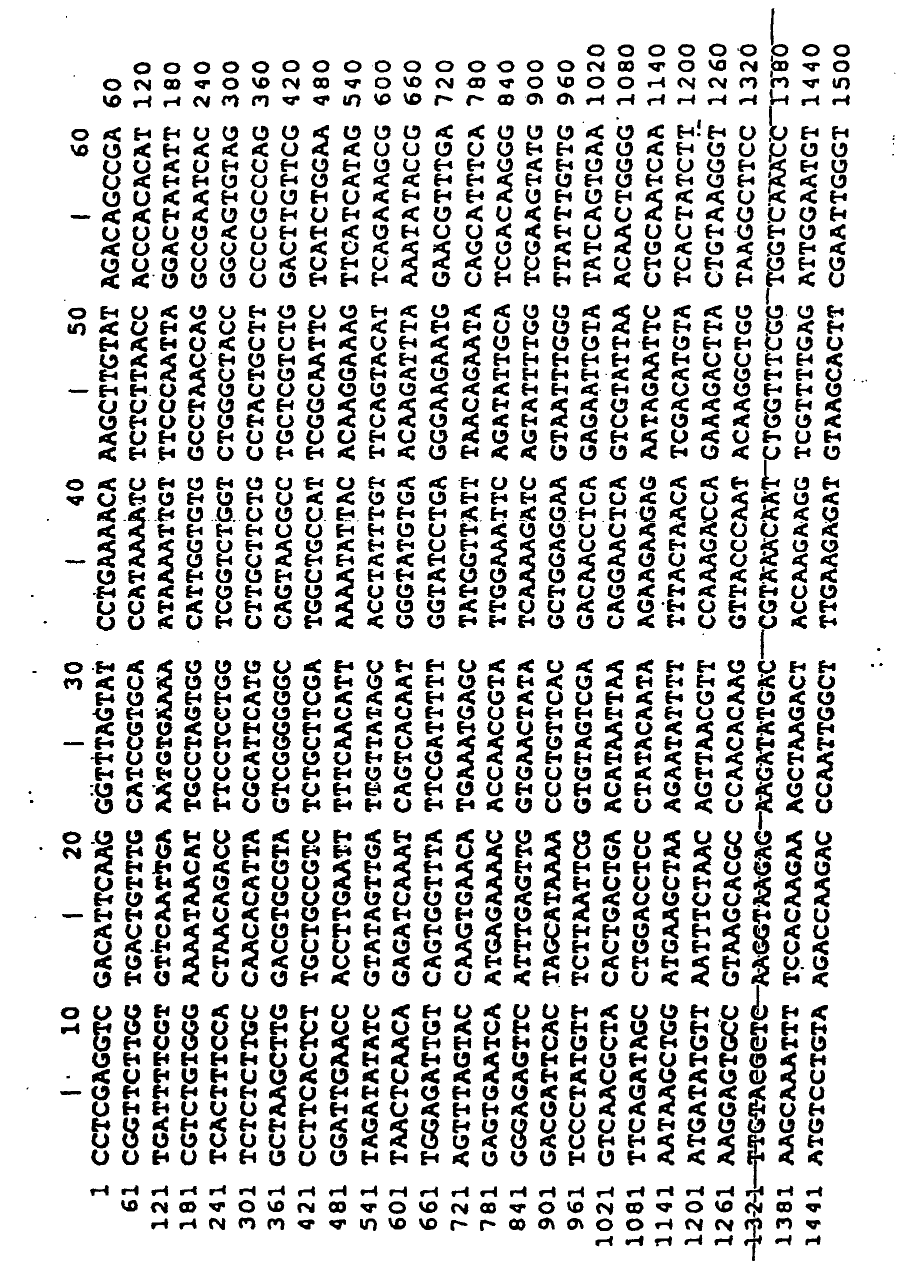 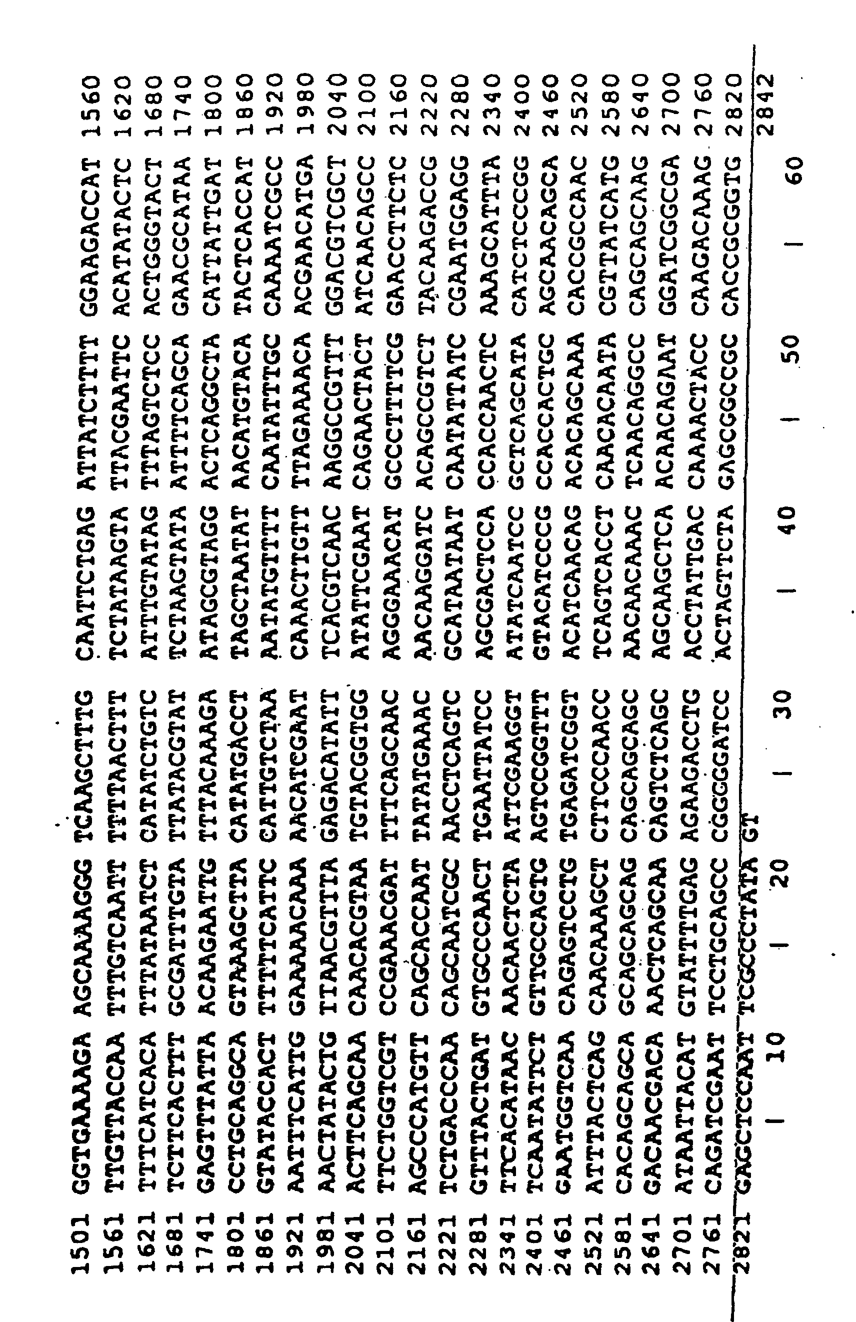 oder dass sie aus der Sequenz II besteht, oder dass sie unter Bedingungen hoher Stringenz mit der komplementären Sequenz zu II hybridisiert, wobei die so gebildete Nukleotidsequenz wenigstens eine der folgenden Eigenschaften besitzt:
- sie kodiert für ein Resistenzprotein gegen Cycloheximid und/oder
- sie kodiert für eine Aminosäureverknüpfung, die geeignet ist, von Antikörpern erkannt zu werden, die gegen das Resistenzprotein gegen Cycloheximid mit der folgenden Sequenz A gerichtet sind: 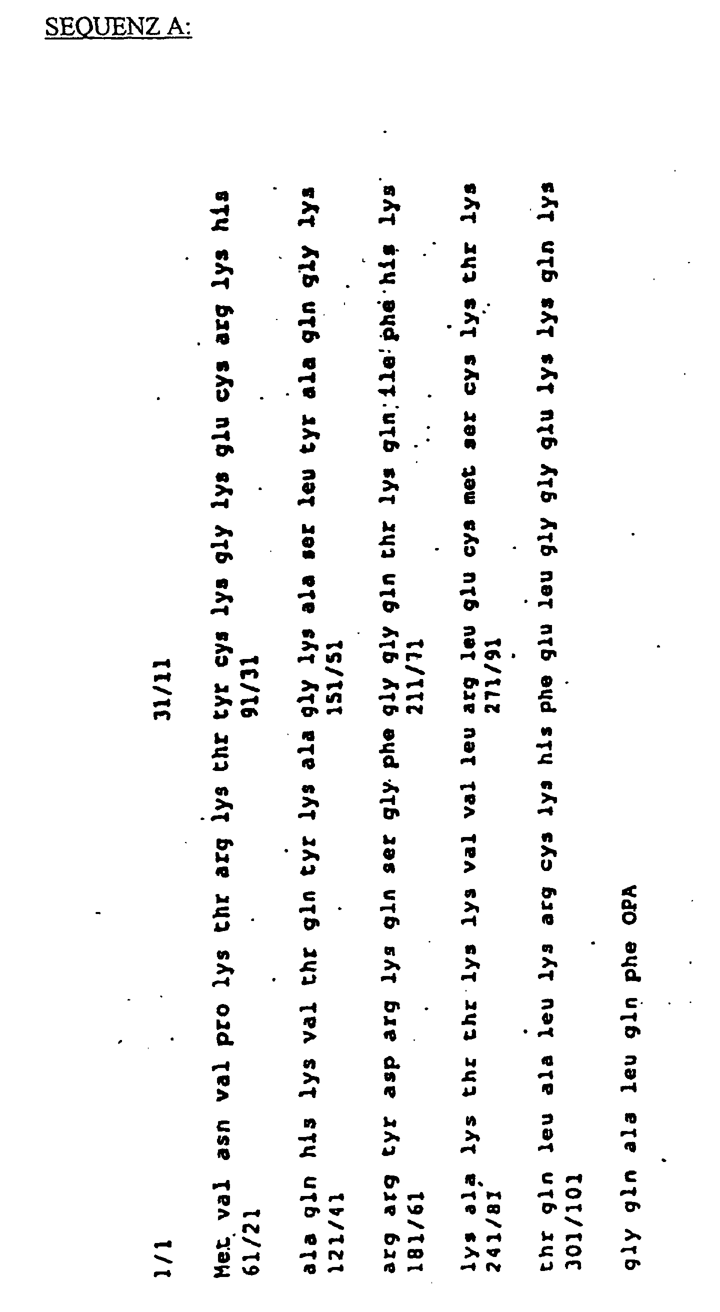 und/oder
- sie reguliert die Expression der für ein Protein nach irgendeinem der Ansprüche 1 bis 4 kodierenden Sequenz in Kluyveromyces lactis.

7. Nukleotidsequenz nach Anspruch 6, **dadurch gekennzeichnet, dass** sie die Nukleotidverknüpfung ORF A enthält, die die Nukleotide 1 bis 1560 der in der Figur 2 dargestellten Verknüpfung umfasst, oder dadurch, dass sie mit der komplementären Sequenz zu ORF A unter stringenten Bedingungen hybridisiert.

8. Nukleotidsequenz nach Anspruch 7, **dadurch gekennzeichnet, dass** sie die folgende kodierende Sequenz I von ORF A enthält:

Sequenz I

AT GGTTAACGTT CCAAAGACCA GAAAGACTTA CTGTAAGGGT
AAGGAGTGCC GTAAGCACGC CCAACACAAG GTTACCCAAT ACAAGGCTGG TAAGGCTTCC
TTGTACGCTC AAGGTAAGAC AAGATATGAC CGTAAACAAT CTGGTTTCGG TGGTCAAACC
AAGCAAATTT TCCACAAGAA AGCTAAGACT ACCAAGAAGG TCGTTTTGAG ATTGGAATGT
ATGTCCTGTA AGACCAAGAC CCAATTGGCT TTGAAGAGAT GTAAGCACTT CGAATTGGGT
GGTGAAAAGA AGCAAAAGGG TCAAGCTTTG CAATTCTGA

9. Nukleotidsequenz nach Anspruch 6, **dadurch gekennzeichnet, dass** sie die Nukleotidverknüpfung III enthält, die die Nukleotide 1 bis 628 der in der Figur 2 dargestellten Verknüpfung oder die in der besagten Nukleotidverknüpfung III enthaltene Promotorregion der Transkription der Sequenz ORF A umfasst.

10. Kofaktor, der geeignet ist, die Resistenz gegen Cycloheximid zu erhöhen, die von einem Protein verliehen wird, das von einer Nukleotidsequenz nach irgendeinem der Ansprüche 7 oder 8 kodiert ist, **dadurch gekennzeichnet, dass** er in der Nukleinsäuresequenz enthalten ist, die zwischen den Nukleotiden 1561 bis 2740 der Verknüpfung enthalten ist, die in Figur 2 dargestellt ist, wobei er es erlaubt, bei einem eukaryontischen Wirt, der mittels Einfügens dieser Nukleinsäuresequenz und der für ein Resistenzprotein kodierenden Nukleotidsequenz verändert ist, unter Bedingungen, die die Produktion dieser Proteine erlauben, eine Höhe der Resistenz gegen Cycloheximid höher als 100 µg/ml zu erreichen.

11. Resistenzprotein gegen Cycloheximid, dadaurch **gekennzeichnet**, dass es aus der folgenden Aminosäureverknüpfung A besteht: 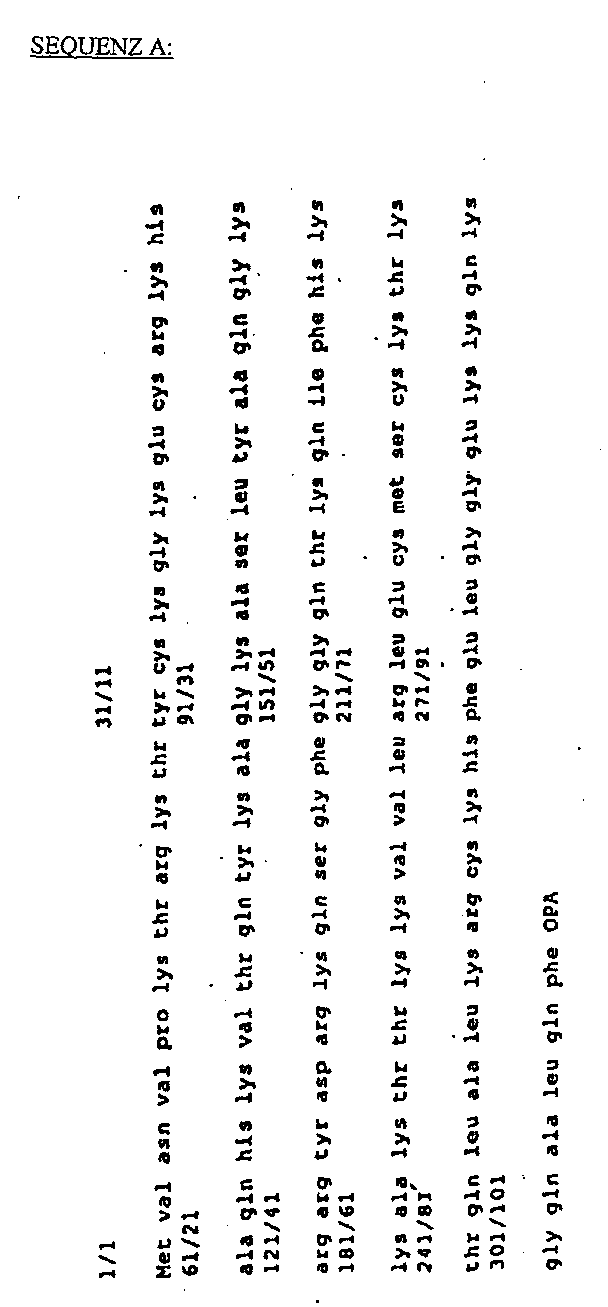 oder dass es die gesamte oder einen Teil dieser gegebenenfalls veränderten Verknüpfung A umfasst, wobei das gebildete Protein einem rekombinanten eukaryontischen Wirt, der mit der für dieses Protein kodierenden Nukleotidsequenz transformiert wurde, unter Bedingungen, die seine Produktion erlauben, eine Resistenz gegen eine Konzentration höher oder gleich 100 µg/ml Cycloheximid verleiht.

12. Resistenzprotein gegen Cycloheximid, **dadurch gekennzeichnet, dass** es von der folgenden Nukleotidsequenz I kodiert ist: 
Sequenz I

AT GGTTAACGTT CCAAAGACCA GAAAGACTTA CTGTAAGGGT
AAGCAGTGCC GTAAGCACGC CCAACACAAG GTTACCCAAT ACAAGGCTGG TAAGGCTTCC
TTGTACGCTC AAGGTAAGAG AAGATATGAC CGTAAACAAT CTGGTTTCGG TGGTCAAACC
AAGCAAATTT TCCACAAGAA AGCTAAGACT ACCAAGAAGG TCGTTTTGAG ATTGGAATGT
ATGTCCTGTA AGACCAAGAC CCAATTGGCT TTGAAGAGAT GTAAGCACTT CGAATTGGGT
GGTGAAAAGA AGCAAAAGGG TCAAGCTTTG CAATTCTGA
 von einem Teil der Sequenz I oder von einer im Vergleich zu I veränderten Sequenz, wobei das von diesem Teil der Sequenz oder von dieser veränderten Sequenz kodierte Protein, wenn es in einen eukaryontischen Wirt eingeführt wird, unter Bedingungen, die seine Expression erlauben, fähig ist, eine Resistenz gegen eine Konzentration höher oder gleich 100 µg/ml Cycloheximid zu verleihen.

13. Protein, das von einer Nukleinssäuresequenz nach irgendeinem der Ansprüche 1 bis 10 kodiert ist, wie sie ausgehend von Kluyveromyces lactis erhalten wird.

14. Rekombinanter Vektor, insbesondere für die Klonierung und/oder die Expression, insbesondere vom Plasmid-Typ, **dadurch gekennzeichnet, dass** er wenigstens eine Nukleotidsequenz nach irgendeinem der Ansprüche 1 bis 10 umfasst, die sich unter der Kontrolle von Regulationselementen befindet, die erforderlich für ihre Expression in einem ausgewählten eukaryontischen Wirt sind, wobei diese Regulationselemente einen gegebenenfalls induzierbaren Promotor und eine Transkriptionsterminationssequenz umfassen.

15. Vektor nach Anspruch 14, **dadurch gekennzeichnet, dass** es sich um einen Vektor, der an die Expression in solchen Hefen wie Pichia pastoris angepasst ist oder um einen an die Expression in einer tierischen Zelle angepassten Vektor handelt, beispielsweise das Baculovirus, oder um einen an die Expression in einer pflanzlichen Zelle angepassten Vektor.

16. Vektor nach irgendeinem der Ansprüche 14 oder 15, **dadurch gekennzeichnet, dass** er außerdem eine bestimmte Nukleinsäure enthält, deren Expression in einem ausgewählten eukaryontischen Wirt man untersucht, wobei diese Nukleinsäure sich unter der Kontrolle von für ihre Expression in dem Wirt erforderlichen Regulationselementen befindet.

17. Vektor nach Anspruch 16, **dadurch gekennzeichnet, dass** die Nukleinsäure sich unter der Kontrolle von Regulationselementen befindet, die die Transkription der Nukleotidsequenzen nach irgendeinem der Ansprüche 1 bis 10 kontrollieren.

18. Eukaryontische Zelle, **dadurch gekennzeichnet, dass** sie mit einem Vektor nach irgendeinem der Ansprüche 14 bis 17 transformiert ist und insbesondere dadurch, dass es sich um eine Hefezelle handelt, beispielsweise Pichia pastoris, um eine tierische Zelle, beispielsweise um eine Insektenzelle oder um eine Säugetier-, insbesondere eine Mäuse- oder Affenzelle, um eine menschliche Zelle oder auch eine pflanzliche Zelle.

19. Rekombinanter Vektor nach Anspruch 14, **dadurch gekennzeichnet, dass** er in dem Stamm E. coli DH5α enthalten ist, der bei der CNCM am 2. Juli 1991 unter der Nummer I-1121 hinterlegt wurde.

20. Verwendung eines Vektors nach irgendeinem der Ansprüche 14 bis 17 in einem Selektionssystem, das in spezifischer Weise auf einen Marker vom Typ Cycloheximid reagiert, in einem eukaryontischen Wirt, insbesondere, um bei diesem Wirt die Aufnahme einer bestimmten heterologen Nukleinsäure zu kontrollieren.

21. Verfahren zur Kontrolle des Vorhandenseins einer heterologen Nukleinsäure in einem zellulären Wirt, **dadurch gekennzeichnet, dass** es umfasst:
- die Transformation des zellulären Wirts mit einem Expressionsvektor, der in Stellen, die nicht essentiell für seine Replikation sind, die heterologe Nukleinsäure umfasst, eine Nukleotidsequenz nach irgendeinem der Ansprüche 1 bis 10, unter der Kontrolle der erforderlichen Regulationselemente für die Expression dieser Sequenzen in dem ausgewählten zellulären Wirt,
- die Kultivierung des so transformierten zellulären Wirts,
- das Inkontaktbringen des Wirts mit einer bestimmten Cycloheximidkonzentration und den Nachweis der Resistenz des Wirts gegenüber diesem Antibiotikum.
